(19)

European Patent Office

Europäisches Patentamt
European Patent Office
Office européen des brevets

(11) **EP 4 398 157 A1**

(12) **EUROPEAN PATENT APPLICATION**

(43) Date of publication:
**10.07.2024 Bulletin 2024/28**

(21) Application number: **23150606.4**

(22) Date of filing: **06.01.2023**

(51) International Patent Classification (IPC):
***G06N 3/088*** (2023.01)     ***G06N 3/045*** (2023.01)
***G16H 50/20*** (2018.01)

(52) Cooperative Patent Classification (CPC):
**G06N 3/088; G06N 3/0455; G06N 3/0895;
G06N 3/09; G06N 3/091; G06N 3/092; G06N 3/096;
G16H 30/40; G16H 50/20;** G06N 3/0464

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Microsoft Technology Licensing LLC
Redmond WA 98052-6399 (US)**

(72) Inventors:
• **OKTAY, Ozan
  Redmond, 98052-6399 (US)**
• **HYLAND, Stephanie
  Redmond, 98052-6399 (US)**
• **BANNUR, Shruthi Jaisimha
  Redmond, 98052-6399 (US)**
• **LIU, Qianchu
  Redmond, 98052-6399 (US)**

• **ILSE, Maximilian
  Redmond, 98052-6399 (US)**
• **Fernando, PEREZ-GARCIA
  Redmond, 98052-6399 (US)**
• **COELHO DE CASTRO, Daniel
  Redmond, 98052-6399 (US)**
• **SHARMA, Harshita
  Redmond, 98052-6399 (US)**
• **BOUZID, Kenza
  Redmond, 98052-6399 (US)**
• **NORI, Aditya
  Redmond, 98052-6399 (US)**
• **ALVAREZ-VALLE, Javier
  Redmond, 98052-6399 (US)**

(74) Representative: **Page White Farrer
Bedford House
21a John Street
London WC1N 2BF (GB)**

(54) **TRAINING A MACHINE LEARNING MODEL BASED ON TEXT AND IMAGE DATA**

(57)     A method comprising: accessing a training data point comprising i) a present image of a target, ii) a prior image of the target captured from an earlier occasion, and iii) and a corresponding textual report on the present and prior images; and training a machine learning model by: a) using an image encoder of the machine learning model, encoding the present image into a present image embedding, and encoding the prior image into a prior image embedding, b) using a difference encoder of the machine learning model, generating a difference embedding representing a difference between the present image embedding and the prior image embedding, c) using a text encoder of the machine learning model, encoding the textual report into a text embedding, d) comparing the text embedding with a temporal image embedding comprising the present image embedding and difference embedding.

Figure 3

**EP 4 398 157 A1**

**Description**

Background

A condition of a human or animal subject (e.g. patient) is often estimated at least in part based on one or more bodily scans, which produce images of the subject's body using imaging technologies such as x-ray, positron emission tomography (PET), magnetic resonance imaging (MRI), ultrasound or microscopy. For example such scans could be used to diagnose an illness of the subject, such as cancer.

Conventionally, a human practitioner (e.g. radiologist) reviews the one or more scans and then manually writes a report giving his or her assessment of the scans. This may include for example a possible diagnosis of one or more conditions which the scan may reveal, and a written explanation of which features in the scan are indicative of the possible diagnosis. For example a radiologist reviewing a chest x-ray might write "A mass is present in the left lower lobe and therefore malignancy must be considered".

More recently machine learning (ML) has been applied to try to improve the quality of such reports. Machine learning is a form of artificial intelligence (AI) which learns to produce a desired output based on training data, using either a supervised, reinforcement or unsupervised approach. For example a common form of ML model is a neural network, often employing a layered approach known as deep learning. Advances in machine learning have enabled automated diagnosis systems that may be used to assist medical professionals and thus improve healthcare workflows, such as to reduce errors and omissions.

In fields such as medicine multi-modal data is often available, e.g. radiology images and corresponding reports. Such data can be used to train a machine learning model to perform automated image analysis. Examples of such models include GLoRIA and ConVIRT. These use image and text encoders to learn to match text to images at a pretraining stage. For example the model may be trained based on medical image scans and corresponding textual reports on the scans written by medical professionals. This enables a form of self-supervised training by encoding and contrasting the images and corresponding text, rather than having to artificially label the data after the fact. Optionally the pretrained encoders can then then also be fine-tuned with labels in a supervised approach.

After training, in the deployment stage, the model can be used to perform image-text retrieval and classification. An object detector or segmentation module is also placed at the output of the image encoder in order to separately detect objects within the images being studied or to segment regions of the images.

Automated image analysis or vision-language processing may also be of interest in other fields, not just medicine, e.g. for performing (at least partially) automated analysis of physical objects, such as diagnostics of mechanical, electronic or electrical devices.

Summary

Reports often contain change information referring to a history of the target, e.g. patient history. However existing self-supervised multi-modal training methods only take into account a scan from a single point in time and the report on that scan, and do not take into account past scans. If the model is trained without reference to the past image context, despite the fact that the corresponding text of the report may contain comments on the change in the target (e.g. improvement or worsening of the patient since the last scan), then the model cannot relate the change information in the report to anything in the image.

It would be desirable to provide a model which can take into account past images so as to make the most out of the existing data, and thus providing a model which is more "data efficient" in that it can extract more information per unit training data and thus provide a better performance for a given number of training cycles (or achieved a comparable performance with existing methods in fewer training cycles).

According to one aspect disclosed herein, there is provided a computer-implemented method of machine learning, the method comprising: accessing a machine learning model; and accessing a training data point comprising i) a present image of a target, ii) a prior image of the target captured from an earlier occasion than the present image, and iii) and a corresponding textual report on the present and prior images. The method further comprises operating the machine learning model to perform encoding operations comprising: a) using an image encoder of the machine learning model, encoding the present image into a present image embedding, and encoding the prior image into a prior image embedding, b) using a difference encoder of the machine learning model, generating a difference embedding representing a difference between the present image embedding and the prior image embedding, and c) using a text encoder of the machine learning model, encoding the textual report into a text embedding. The method further comprises training the machine learning model by comparing the text embedding with a temporal image embedding comprising the present image embedding and difference embedding.

The prior image may have been captured, for example, more than one hour prior to the present image, more than one day prior to the present image, more than one week prior to the present image, or more than one month prior to the present image. In embodiments the present and prior images are separate still images and not part of a same video file as one another.

The comparing may be performed by evaluating a similarity metric such as a contrastive loss metric.

In addition to the greater data efficiency, the disclosed method also enables the model to become more aware of temporal language in reports compared to existing methods. Once trained the model can thus be used to generate reports which capture the change information.

Hence in embodiments, The method further comprises, in a deployment stage following said training: receiving a query comprising at least a respective present image and a draft report comprising text describing a target shown in the respective present image; inputting the queried image into the image encoder, thereby generating a respective present image embedding; inputting at least some of the text of the draft report into the text encoder, thereby generating a respective text embedding; based on the respective present image embedding and text embedding of the query, generating a proposed update to the text of the draft report; and outputting the proposed update to a user, thereby causing the user to action a treatment on the target based on the proposed update.

For example, the additional temporal cue help better understand the condition of the patient or other such target, even in the static case (i.e. even if the query comprises only one image), because the model has learned more granularity in the latent space. For instance even if at deployment time the query comprises only a single scan of a patient, the model has learned from the training stage what the condition in question (e.g. pneumonia) looks like at different stages of its development and hence can potentially recognize the present stage of the condition in the current image of the patient in the query at the deployment stage.

Hence in embodiments the query does not comprise any prior image captured from an earlier time than the respective present image

In embodiments the method may further comprise: accessing at least one partial data point comprising respective textual report and a respective present image but no prior image; performing further encoding operations comprising: - using the image encoder to encode the respective present image of the partial data point into a respective present image embedding, - using a missing image encoder of the machine learning model to generate a missing image embedding as a placeholder in lieu of a respective difference embedding, and - using the text encoder to encode the respective textual report of the partial data point into a text embedding; and training the machine learning model by comparing the respective text embedding of the partial data point with the respective present image embedding and with the missing image embedding, wherein the training comprises training the missing image encoder to learn to generate missing image embeddings to act as a placeholders for missing prior images.

As a further alternative or additional feature, in embodiments the difference encoder may be implemented using a vision transformer to avoid the need for image registration between the present and prior images.

As yet another alternative or additional feature, in embodiments at least some of the training may employ masked language modelling (MLM) to the text combined with information from the image domain to improve the robustness of the model.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Nor is the claimed subject matter limited to implementations that solve any or all of the disadvantages noted herein.

Brief Description of the Drawings

To assist understanding of the present disclosure and to show how embodiments may be put into effect, reference is made by way of example to the accompanying drawings in which:

Figure 1 is a schematic block diagram of a system for scanning subjects and generating reports in accordance with embodiments disclosed herein,

Figure 2A is a flow chart of a method of training a machine learning model,

Figure 2B is a flow chart of a method of using a trained machine learning model in a deployment stage,

Figure 2C is a flow chart of a method of generating reports,

Figure 3 schematically illustrates methods of training a machine learning model based on text and image data,

Figure 4 is a schematic illustration of a machine learning model according to one aspect disclosed herein, and

Figure 5 schematically illustrates an optional extension to the model for making predictions in accordance with embodiments disclosed herein.

Detailed Description of Embodiments

The present disclosure provides a method and machine leaning model for accounting for temporal information in vision-language processing, such as biomedical vision-language processing.

For example, evaluating the trajectory of a patient's health is paramount for clinical decision-making, which takes into account prior medical scans and patient history and is an essential feature for decision support systems. Nevertheless, prior work on multi-modal learning for radiology applications has largely focused on the analysis of single radiology studies and omitted the temporal information stored in clinical notes. Utilising such rich multi-modal information in self-supervision can enable a wide range of new downstream applications (e.g., disease progression) whilst further reducing the data dependency of models for supervised tasks. In this work, we present an image model and a self-supervised, multi-modal pre-training approach for image and language biomedical data, accounting for temporal information. It enables both the analysis of single images, as in prior work, as well as pairs of images where prior studies are available. Importantly, the proposed approach improves temporal progression prediction and report generation results over prior baselines and achieves state-of-the-art performance for single image tasks by greatly reducing the need for labelled data.

The present disclose provides a novel deep learning technique to process longitudinal medical image to enable precision medicine. The disclosed method can learn from historical medical data in an unsupervised manner without requiring expert labels whilst achieving state-of-the-art downstream predictive performance.

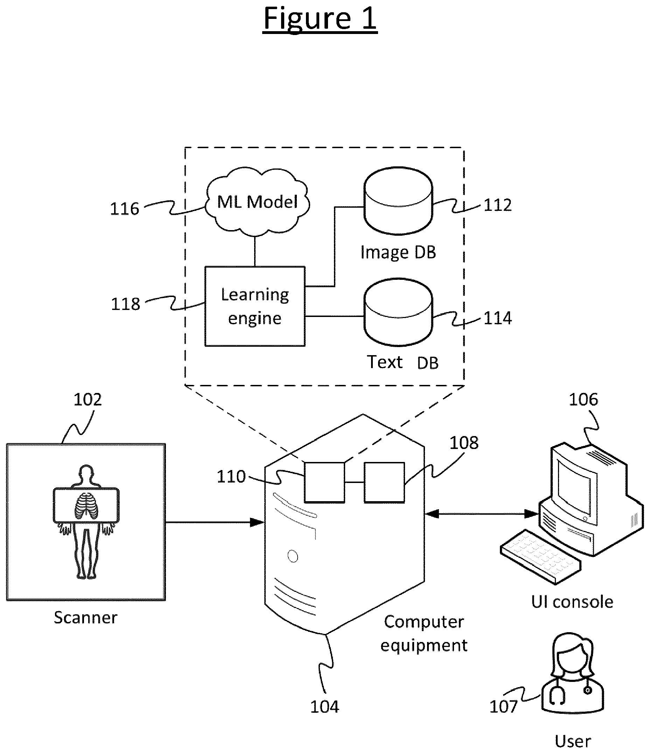

Figure 1 shows an example system in accordance with embodiments of the present disclosure. The system comprises at least one image source (e.g. image capturing device) 102, computer equipment 104, and at least one user interface (UI) console 106. The at least one image source 102 and the UI console 106 are operatively coupled to the computer equipment 104, either directly or via one or more networks, such as the Internet, a mobile cellular network, and/or a private wired or wireless intranet of an organization (e.g. hospital, health organization or research institute). It will be appreciated that any data, signals or information described below as being exchanged between the image source 102 and the computer equipment 104, and between the computer equipment 104 and the UI console 106, may be conducted by any such means of connection and/or others.

The at least one image source 102 may comprise a scanner for producing scans of at least a bodily part of a human or animal subject, living or dead, based on one or more scanning media types. For example the at least one scanner 102 may comprise an x-ray based scanner for producing x-ray scans, a PET scanner for producing PET scans, an MRI scanner for producing MRI scans, an ultrasound scanner for producing ultrasound scans, and/or scanning electron microscopy equipment for producing electron microscopy based scans. Another example could be visible light photography equipment for producing scans in the form of photographs. In embodiments the system may comprise multiple types of scanner for producing multiple types of scan of a given subject based on different scanning media (e.g. x-ray and MRI, x-ray and PET, PET and MRI, etc.). Alternatively only a single scanner employing a single type of scanning medium may be used, e.g. x-ray only, or MRI only, etc. Note also that for a given scanning medium type, there may be different options from producing an image. For example an x-ray based scan as referred to herein could refer to an x-ray photograph or a more sophisticated form of scan based on x-rays as the probing medium, such as a CT (computed tomography) scan. Or a visible light based scan could refer to a simple macroscopic visible light photograph or an optical microscopy based scan.

The following will be described in terms of vision-language processing of bodily scans obtained by an image source 102 in the form of an image scanner such as a medical scanner. However it will be appreciated that this is not limiting. In other use-cases, the images could be any images from any image source 102, such as one or more cameras or an image database of another system. Any mention of scans in the Detailed Description could be replaced more generally with any set of one or more images, and the reports could comprise any pieces of text describing the presence or absence of any being, object or phenomenon in the corresponding images.

In the case of bodily scans or the like, each individual scan may comprise one or more images. In the case of multiple images per scan, the scan may for example comprise a 3D "stack" of 2D images ("slices"), each image comprising a cross-section through the subject taken at a different respective depth. For instance, while a simple x-ray photograph may comprise only a single x-ray image, a more sophisticated x-ray technology such as a CT (computed tomography) scan may comprise a stack of images representing slices through the subject at different depths, thus creating a tack of 2D images representing a 3D volume of at least a portion of the subject's body. The scanning equipment 102 may be used to take a 2D or 3D scan of a given subject on a single occasion or multiple such scans taken on multiple different respective occasions (different times), e.g. spaced apart by hours, days, weeks, months or years.

The computer equipment 104 may comprise a single computer unit in a single housing, or multiple units in separate housings at one or more sites and networked together using any suitable network technology. For example the computer equipment 104 may comprise one or more server units. E.g. the computer equipment 104 may comprise multiple server units in the same rack, or different units or racks in the same room, different units or racks in different rooms of the same facility or data centre, and/or different facilities or data centres at different geographic sites. In the case of multiple computer units (e.g. server units), these may for example be networked together via a server area network, local area network, intranet, campus area network, metropolitan area network and/or a wide-area network or internetwork such as a mobile cellular network and/or the internet.

The UI console 106 may be integrated into one or more of the computer units of the computer equipment 104, and/or may comprise one or more separate computer terminals or dedicated UI peripherals. The UI console 106 comprises at least one display screen, and at least one form of user input means (e.g. a touch screen and/or point-and click interface of the display, and/or a keyboard or voice interface). The UI console is thus able to provide a user interface (UI) for outputting the images of the scans to a user 107, receiving text from the user 107 for writing reports based on the images, and displaying the text of the reports and associated suggestions generated by the computer equipment 104 based on the original text and image(s). The user 107 may comprise for example a medical practitioner, veterinarian, or a researcher. The user 107 may comprise a single such person or a team of two or more people.

The computer equipment 104 comprises processing apparatus 108 comprising one or more processors, and memory 110 comprising one or more memory units. The processor, or each processor, may take any suitable form such as a CPU (central processing unit), or a dedicated AI accelerator processor, or some other, re-purposed form of application-specific processor such as a GPU (graphics processing unit), crypto-processor or DSP (digital signal processor). The memory unit, or each memory unit, may take any suitable form, e.g. an electronic memory medium such as a SSD (solid state drive), flash memory, ROM (read only memory), EEPROM (electrically erasable and programable ROM), RAM (random access memory), SRAM (static RAM), and/or DRAM (dynamic RAM); and/or a magnetic memory such as a hard drive, removable magnetic disk, and/or magnetic tape drive; and/or optical memory such as CD (compact disc), DVD (digital versatile disk), other optical disk, quartz glass storage, and/or magneto-optical drive; and/or synthetic biological storage such as synthetic DNA storage.

In some examples, computer executable instructions are provided using any computer-readable media that are accessible by the computing equipment 104. Computer-readable media include, for example, computer storage media such as memory 110 and communications media. Computer storage media include volatile and non-volatile, removable, and non-removable media implemented in any method or technology for storage of information such as computer readable instructions, data structures, program modules or the like. Computer storage media include, but are not limited to, Random Access Memory (RAM), Read-Only Memory (ROM), Erasable Programmable Read-Only Memory (EPROM), Electrically Erasable Programmable Read-Only Memory (EEPROM), persistent memory, phase change memory, flash memory or other memory technology, Compact Disk Read-Only Memory (CD-ROM), digital versatile disks (DVD) or other optical storage, magnetic cassettes, magnetic tape, magnetic disk storage, shingled disk storage or other magnetic storage devices, or any other non-transmission medium that can be used to store information for access by a computing apparatus. In contrast, communication media may embody computer readable instructions, data structures, program modules, or the like in a modulated data signal, such as a carrier wave, or other transport mechanism. As defined herein, computer storage media do not include communication media. Therefore, a computer storage medium should not be interpreted to be a propagating signal per se. Propagated signals per se are not examples of computer storage media. Although the computer storage medium (the memory 110) is shown within the computing equipment 104, it will be appreciated by a person skilled in the art, that, in some examples, the storage is distributed or located remotely and accessed via a network or other communication link (e.g., using a communication interface).

Whatever form it takes, the memory 110 is arranged to store an image database 112, a text database 114, a machine learning model 116, and a learning engine 118. The image database stores the image data from one or more scans taken by the at least one scanner 102. It may also store one or more past or historic scans which may have been taken via the same scanning equipment 102 or other scanners, or a combination. The text database 114 stores text data, such as a reports on the one or more scans taken by the scanner 102, e.g. the reports having been authored by the user 107 via the UI console 106. The text database 114 may also store one or more past or historic reports authored based on the past or historic scans, which may have been authored via the UI of the same UI console 106 or a different UI, by the same user 107 or a different user, or a combination. Alternatively or additionally, the text database 114 may also store other textual training data such as past medical articles or papers, etc., or more generally any text passages comprising suitable vocabulary for learning the language of the domain in question.

The machine learning (ML) model 116 may comprise one or more neural networks, or any other form of statistical model capable of learning based on training data, such as a clustering model. The machine learning engine 118 comprises software arranged to take one or more scans from the image database 112 and one or more associated reports from the report database, and input them to the machine learning model 116 to cause it to make predictions (also called inferences) based on the input image and report data. Particularly, these predictions comprise one or more suggested updates to the report(s), as will be discussed in more detail shortly. The machine learning engine 118 may also comprise one or more machine learning algorithms arranged to train the machine learning model 116 based on past images and associated reports from the image database 112 and report database 114 respectively. Alternatively the model may have been trained elsewhere, on other computer equipment (not shown), and copied to the memory 110.

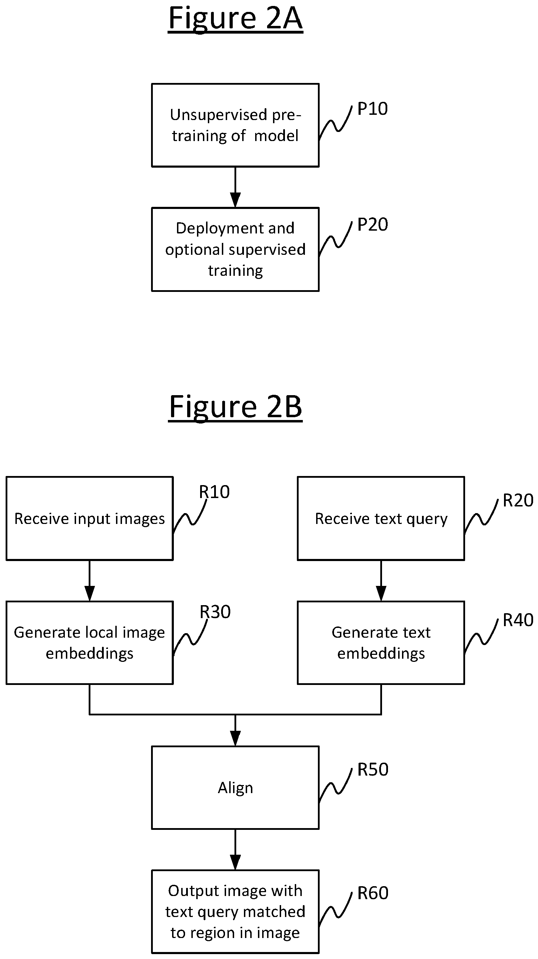

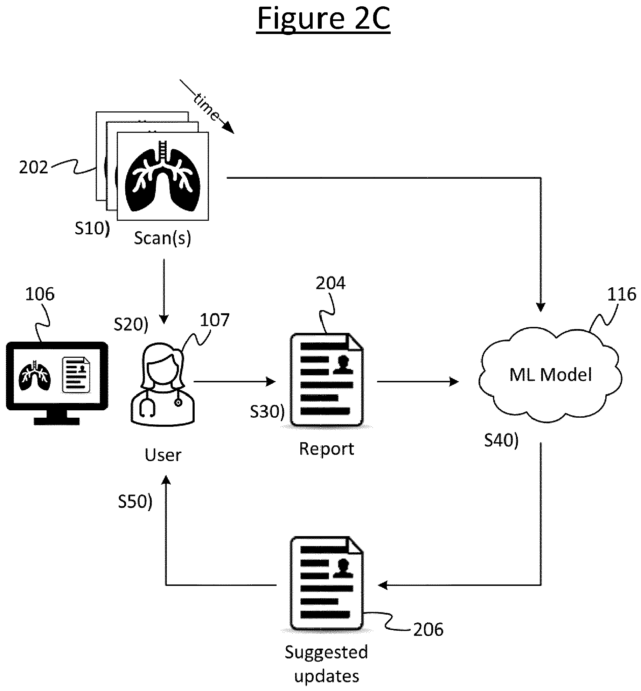

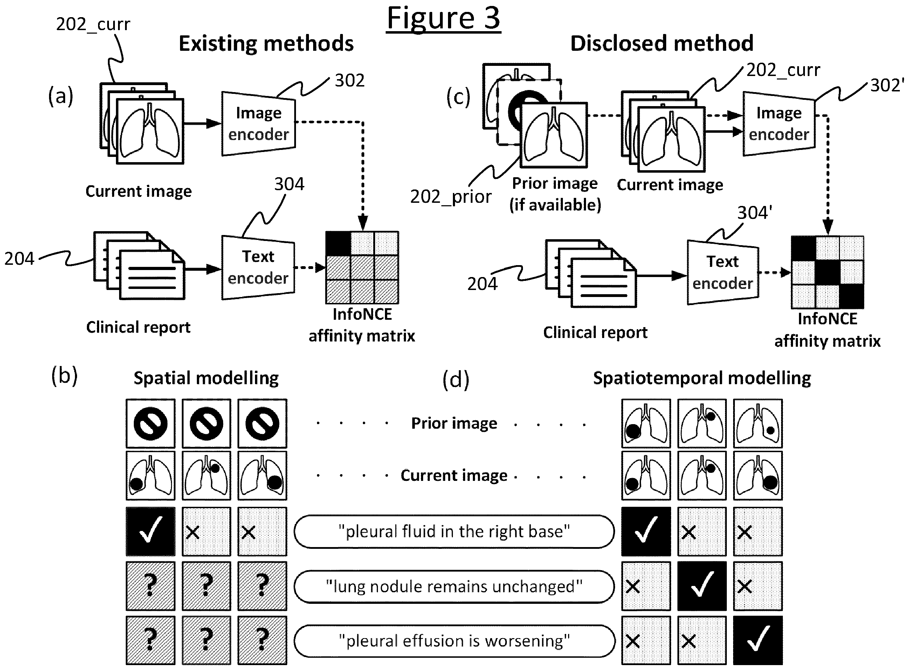

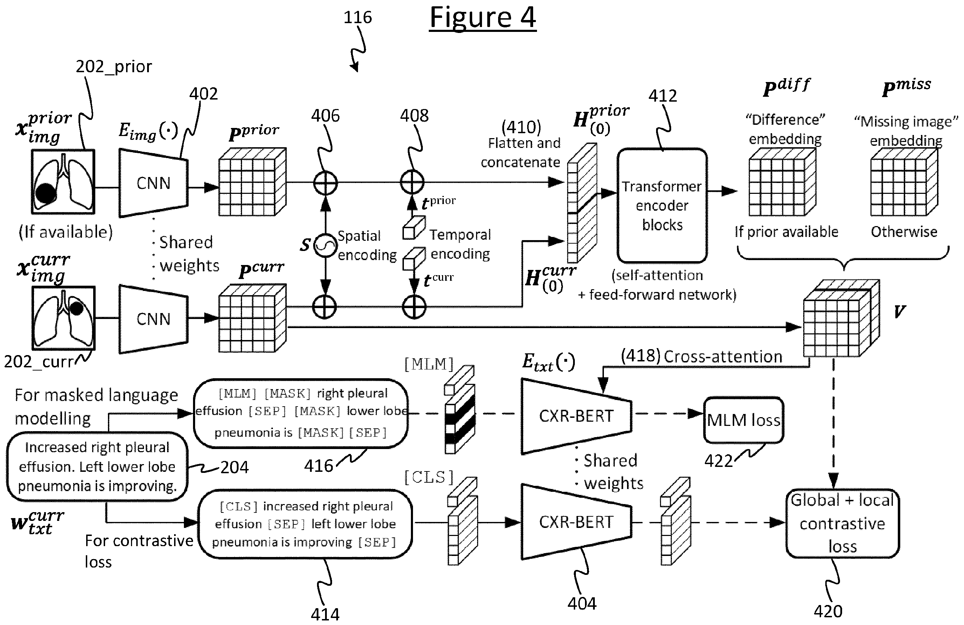

Figure 2A shows an overall method of training and using the ML model 116. At step P10 the method comprises performing an unsupervised pre-training of the ML model 116 under control of the ML engine 118. The unsupervised pre-training is based on multi-modal data comprising image data (e.g. records of scans) from the image database 112, and text data in the form of textual reports from the text database 114, rather than requiring explicitly labelled image data. This unsupervised training will be discussed in more detail shortly with reference to Figures 3 and 4. At step P20 the method then proceeds to a deployment stage where the ML model 116 is used for making predictions. Optionally, this stage may also comprise further, supervised training of the ML model 116 based on user feedback on the predictions from the user 107, or based on a previously acquired set of manual labels. Methods of using the ML model 116 following the initial unsupervised pre-training are discussed in more detail later with reference to Figures 2B and 2C.

Figure 3 shows a method of unsupervised multi-modal training in accordance with embodiments of the present disclosure, contrasted against existing methods.

As shown in Figure 3(a), the ML model 116 comprises an image encoder network 302 and a text encoder network 304. In existing methods, the image encoder network 302 is used to encode current image (e.g. medical scan) 202_curr into an image embedding, and the text encoder network 304 is used to encode a corresponding textual report 204 into a text embedding. The report 204 is a report on the image (e.g. scan), for example having been prepared by a medical professional to diagnose a condition visible in the scan, so the report 204 contains information which can be used for self-supervised training without separate labelling of the image 202. Accordingly, the method then evaluates a similarity metric such as a contrastive loss metric representing a difference between the image and text embeddings. This is repeated over multiple image-text pairs and the encoder networks 302, 304 are trained based on the evaluated metric. E.g. the image and text encoder networks 302, 304 may each comprise a neural network and the training comprises tuning the weights of the neural networks. The image embedding may comprise a matrix of constituent patch embeddings, each encoded from a different respective patch (i.e. spatial sub region) of the image. The text embedding may also comprise more than one constituent embedding, each encoded from a different potion (e.g. a different sentence or clause) of the textual report 204. In this case the evaluation of the similarity metric may comprise evaluating a local similarity between each possible combination of text-portion embedding (e.g. sentence embedding) from the report 204 and patch from the image 202 - i.e. comparing each constituent text-portion embedding with each patch embedding - to thus determine an affinity matrix. The encoder networks 302, 304 of the model 116 are then trained to find a mapping - a so-called "alignment" - between text-portion embeddings and corresponding patch embeddings which maximises the similarity metric for the corresponding aligned patches. Similarity metrics and methods for forming an affinity matrix and training encoders based thereon are, in themselves, known in the art. One example is the InfoNCE (noise contrastive loss) function.

The elements of the grid representing the affinity matrix as shown in Figure 3(a) and Figure3(c) can correspond to patch-sentence instances (or patch - text-portion instances), i.e. individual pairings of report text portion with a most aligned image patch; but they can also be defined as image-report pair instances, i.e. pairings of a whole report to a whole image. The latter is what is illustrated by way of example in Figures 3(a) and 3(c). The pairing of image-text data can be customised depending on the granularity of information that one is interested in learning. In general, a measure of local and/or global similarity may be used in the learning objective.

The connection between InfoNCE (contrastive loss) and the affinity matrix can be explained as follows. Imagine that a large set of paired image and text data samples obtained from N different patients. At training time, these samples are provided without pairs, as it is not desired to reveal to model which images samples belong which text reports. The model needs to estimate the true pairs and false pairs by analysing both data modalities. This can be now visualised as an affinity matrix where there are N rows of sentences/reports and N columns of images, wherein for visualization purposes the rows and columns can be imagined as sorted such that the diagonal elements correspond to the true pairs. The InfoNCE objective rewards the model if the model identifies these correct true pairs (diagonal elements) by assigning high similarity scores compared to the off-diagonal elements that represent the negative/false pairs. An additional remark is that without having access to prior images and their embeddings, the model will not be able to make this distinction between true/false pairs if text mentions change information. Therefore, enforcing an InfoNCE loss/reward mechanism becomes sub-optimal.

As shown in Figure 3(b), the existing methods only use a current image 202_curr and the report 204 on that image (e.g. scan). However, the report 204 could contain temporal statements describing how what is found in the current image 202_curr compares to a prior image 202_prior that was captured at an earlier time, e.g. "lung nodule remains unchanged". Existing methods will not be able to match the embeddings for such statements to anything in the image 202_curr as it does not contain historical information.

Figure 3(c) shows an upgraded method in accordance with the present disclosure, which may be performed under control of the ML engine 118. The ML model 116 again comprises an image encoder network 302' and a text encoder network 304'. However the image encoder network 302' is arranged to receive both a current (i.e. present) image 202_curr and at least one prior (i.e. previous) image 202_prior which shows at least part of a same target being, object or phenomenon (e.g. patient body part or condition) as the current image 202_curr. These may be received from the image database 112. Each of the current image 202_curr and the prior image 202_prior is a static image, and they are captured on different occasions from one another (e.g. more than one hour, day, week or month apart). Each of the current image 202_curr and prior image 202_prior may be a 2D image or a 3D image (e.g. volumetric scan). For example the current and prior images 202_curr, 202_prior may for example be different medical scans of the target captured from different scan events, using any of the modalities discussed earlier. As will be discussed later, in embodiments the model can make use of multiple prior images of the target, but for now the principle will be discussed with reference to one prior image 202_prior. The current and prior images, or the multiple prior images, may have been captured using the same imaging modality as one another, or different modalities (e.g. one could be an MRI scan and another could be an x-ray photograph or CT scan). The current and prior images, or the multiple prior images, could be taken from the same angle as one another relative to the target, or from different angles (e.g. frontal and lateral images). Note also that "current" or "present" as used herein does not necessarily imply an image from the immediate point in time at which the method is performed, but rather the latest in a series of two or more images being used as a data point for training or in a query. Nor do these terms exclude that there could be a subsequent image that is used to update the training or make a new query later on.

The image encoder network 302' is arranged to encode the current image 202_curr into a current image embedding and to encode the prior image 202_prior into a prior image embedding. Preferably a shared network is used for both, but alternatively it is not excluded that the image encoder network 302' could comprise two separate subnetworks, one used to encode the current image 202_curr and one used to encode the prior image 202_prior. Either way, the image encoder network 302' also compromises an additional part configured to generate a difference signal representing a difference between the present and past images 202_curr, 202_prior. The two image representations are later combined into a combined temporal image embedding capturing both the current image content and also the signal representing difference (e.g. disease progression). This is what's represented by $V$ in Figure 4 (discussed in more detail later).

As in the existing case, the text encoder network 304' receives a corresponding textual report 204 (e.g. from text database 114) and is used to encode the corresponding textual report into a text embedding. The text encoder 304' may be arranged in a similar manner to the exiting case of Figure 3(a), or may have additional modifications applied as will be discussed in more detail shortly.

A similarity metric such as a contrastive loss metric is then evaluated to compare the text embedding with the combined image representation $V$ (comprising both the current image embeddings and the difference embeddings), and the encoder networks 302', 304' of the ML model 116 are trained based on this over a plurality of data points. In embodiments the difference signal comprises a per-patch difference embedding, and this is used to determine an affinity matrix between the combined temporal image representation $V$ and the text-portion embeddings. That is, a single image representation is formed for each image patch (See $V$ in Figure 4, to be discussed in more detail later), and this representation comprises both the current image information ($P^{curr}$) and also the difference information across time ($P^{diff}$). In other words, if the grid of current image is H × W number of patches, where H and W being height and weight, then a single representation is generated for each patch in this grid (HxW) that is aware of both the current content and how things have evolved compared to the past scans. Once $V$ is computed, then the usual infoNCE based training is performed as shown in Fig 3(d); thus there is only a single affinity matrix since the disclosed image encoder can encapsulate both information into fused representation. This can later be used to identify the correct report or sentence regardless of its static or temporal content.

As illustrated in Figure 3(d), the disclosed method therefore enables temporal statements in the report to be matched to information from past images 202_prior, and thus the model 116 can be trained to recognise and extract information from such statements.

Figure 4 shows an example implementation of the disclosed model in more detail. This may be implemented as an upgraded version of the ML model 116 shown in Figure 1, and trained under control of the ML engine 118.

The ML model 116 comprises an image encoder 402, which may also be denoted $E_{img}(\cdot)$. This may take the form of a neural network such as a convolutional neural network (CNN). Alternatively it is not excluded that the image encoder could comprise another form of trainable statistical model such as a decision forest or clustering model, or a combination of model types. The ML model 116 also comprises a difference encoder 412. The image encoder 402 and difference encoder 412 correspond to the image encoder network 302' shown in Figure 3. In embodiments the difference encoder 412 comprises a vision transformer, which is a known type of modern image encoder that can capture image differences on a local (i.e. patch) level without relying on image registration or spatial aligninment of the images. However in alternative embodiments it is not excluded that the difference encoder could be implemented as another neural network or other type of model.

The ML model 116 further comprises a text encoder 404, and optionally a masked language modelling (MLM) loss function 422. The text encoder 404 and optional MLM loss function 420 correspond to the text encoder network 304' of Figure 3.

In operation during an unsupervised training stage, the ML model 116 is arranged to receive a training data point comprising: i) a current (i.e. present) image 202_curr, ii) at least one corresponding prior (i.e. previous) image 202_prior, and iii) and corresponding a textual report 204 (i.e. a report comprising text). The present image may also be denoted $x_{img}^{curr}$, the prior image may also be denoted $x_{img}^{prior}$, and the text report may also be denoted $w_{txt}^{curr}$. The current and prior images 202_cur, 202_prior may each be a 2D or 3D image. They are preferably separate still images captured by an order of magnitude of more than one hour, one day, one week or one month apart, and not frames of a video which are typically captured at multiple frames per second. The current and prior images 202_curr, 202_prior may be received from the image database 112 and the report 204 may be received from the text database 114. The current image 202_curr shows a certain target, which could comprise a person or animal, or a body part thereof, or any other object or phenomenon depending on the application context. For a given data point, the prior image 202_prior contains image information on at least part of the same target as the corresponding current image 202_curr, and the report 204 comprises text describing a condition of the target (e.g. estimated presence, absence, degree or likelihood of a certain disease) as seen in the present and past images 202_cur, 202_prior. The text of the report has been authored by a professional in the relevant field, e.g. medical professional, reviewing the present and past images. Alternatively it is not excluded that some of the text could have been generated based on an automated process.

Note: the disclosed encoding strategy is not limited only to two images (present and one past image) but can be linearly scaled to any arbitrary number of past images, in embodiments via using cross-attention in the vision transformer block. Figure 3(c) and 4, and the description thereof, visualize and explain the case of two images (a current image and one past image) for the sake simplicity, and indeed certain applications may not require more than one prior image. However, if the reports describe a history going back over a period covered by more than one image, e.g. patient history going back over a period for which more than one past scan is available, then there may be value in processing more than two scans or images (one current image and more than one past image). The following will be described in terms of one past image by way of illustration, but it will be appreciated that this is not limiting.

The current image 202_curr is input into the image encoder 402 to thereby generate a respective current image embedding $P^{curr}$. The prior image 202_prior is input into the image encoder 402 to thereby generate a respective prior image embedding $P^{prior}$. In embodiments each image is divided into a plurality of patches, i.e. spatial subdivisions, e.g. a in a grid, and the image encoder 402 is configured to encode each patch of each image into a respective patch embedding. In this case the current image embedding $P^{curr}$ therefore comprises a plurality (e.g. matrix) of constituent current patch embeddings, each encoded from a different respective patch of the current image 202_curr; and the prior image embedding $P^{prior}$ comprises a plurality (e.g. matrix) of constituent prior patch embeddings, each encoded from a different respective patch of the prior image 202_prior.

In embodiments the image encoder 402 comprises a shared neural network, comprising some or all of the same weights, for encoding both the current image 202_curr and the past image 202_prior. This is more efficient than using separate neural networks and separate networks are not necessary as there is not necessarily any reason to believe that a past image should be encoded any differently than a present image for identifying key information (e.g. clinical findings). Nonetheless it is not excluded that in other embodiments the image encoder 402 could comprise a first subnetwork arranged to encode the current image 202_curr of a given data point, and a separate second subnetwork, having independently tuneable weights, arranged to encode the corresponding prior image 202_prior.

The current image embedding $P^{curr}$ and prior image embedding $P^{prior}$ are input to the difference encoder 412. They may be flattened (i.e. converted to 1D) and concatenated (410) prior to input to the difference encoder 412. Based on the current image embedding $P^{curr}$ and prior image embedding $P^{prior}$, the difference encoder 412 generates a difference embedding $P^{diff}$ representing a difference between the current image embedding $P^{curr}$ and prior image embedding $P^{prior}$. In embodiments where these comprising a plurality of constituent patch embeddings, the difference embedding $P^{diff}$ may also comprise a plurality of constituent difference patch embeddings corresponding to different patches of the image. Note: there is not necessarily a direct mapping between a given difference patch embedding and co-located embeddings in both present and prior images. Rather, for a given patch in current image, the transformer performs a comparison across all the patches available in the prior images, extracts a fused representations from patches in the prior image, and finally merges this new feature content with the patch embedding in current image. From this perspective, the two grids (present image and prior image) do not have to be the same. However, the grid of the difference embeddings and current image patch embeddings will preferably be the same, so as to be able to combine them into a single grid of final representation (**V**).

In embodiments, the difference encoder 412 comprises a vision transformer. A vision transformer, in itself, is a known form of difference encoder which avoids the need for image registration, i.e. transforming one or both of two images so that the feature(s) in the image are exactly aligned in the same image plane. This is advantageous as, for example, two different medical scans might not be taken from exactly the same angle relative to the patient's body (e.g. because the patient did not lie or sit in exactly the same angle or position for both scans), such that if a direct patch-wise comparison was performed between each patch in the current image and its corresponding collocated patch in the prior image, then the model 116 would not necessarily be strictly comparing the same feature like-for-like. Applying image registration is not necessarily desirable as it can be processor intensive and is difficult to get accurate. However in alternative embodiments, instead of a vision transformer, it is not excluded that image registration could be applied combined with an alternative form of difference encoder such as a conventional neural network.

In the case of a vision transformer, then according to the present disclosure each of the current and prior image embeddings $P^{curr}$, $P^{prior}$ is encoded into a spatially and temporally encoded version $H^{curr}_{(0)}, H^{prior}_{(0)}$. That is, each constituent patch embedding of the current image embedding $P^{curr}$ is combined 406 (e.g. concatenated or multiplied) with a respective spatial encoding $S$, and the current image embedding $P^{curr}$ as a whole is combined (e.g. concatenated or multiplied) with a respective temporal encoding $t^{curr}$. Similarly each constituent patch embedding of the prior image embedding $P^{prior}$ is combined (e.g. concatenated or multiplied) with the respective spatial encoding S for the respective patch, and the prior image embedding $P^{curr}$ as a whole is combined 408 (e.g. concatenated or multiplied) with a respective temporal encoding $t^{prior}$. The spatial encoding for each patch is an identifier of the spatial position of each patch. It is a fixed value for a given patch. E.g. the spatial encoding may be a sinusoidal spatial encoding. The temporal encoding is an identifier of the time of an image. It is a fixed value for a given point in time, or always the same for the current image and always the same for the prior image (but has a different for the current and prior images). The current and prior image embeddings $P^{curr}$, $P^{prior}$ are then input into the vision transformer in their spatially and temporally encoded form $H^{curr}_{(0)}, H^{prior}_{(0)}$ and the vision transformer computes the difference embeddings $P^{diff}$ based on these. The spatial encoding distinguishes between different patches and the temporal encoding distinguishes between the present and past image 202_curr, 202_prior. This provides a hybrid version of a conventional vision transformer which would conventionally not retain any spatial grid information. According to embodiments of the present disclosure on the other hand, the spatial encoding 5 forces the vision transformer 412 to retain some concept of spatial position, in terms of patches, whilst still avoiding the need for image registration. The temporal encoding $t^{curr}$, $t^{diff}$ ensures a distinction between the present and past images. The temporal encoding gives the model a concept of direction across time. If there was no temporal encoding, then the model would output the same difference features regardless of swapping the order of present and prior images. By having a sense of temporal direction, the model can distinguish whether a clinical finding is improving or worsening as time progresses. Without this, it would only be able to say that it's changing but not the direction.

Embodiments thus employ a hybrid ML model 116 comprising a convolutional neural network (CNN) for the image and text encoding 402, 404 and a vision transformer 412 for the difference encoder. A vision transformer on its own needs a large amount of data to train, whereas the disclosed hybrid approach can work based on less data due to the inductive bias of the CNNs.

The matrix $V$ in Figure 4 represents the final image representation in latent space, made up of the current image embedding $P^{curr}$ and the difference embedding $P^{diff}$. Note how the current image embedding $P^{curr}$ and the difference embedding $P^{diff}$ are kept as two separate parts of the image vector $V$ in the latent space. This ensures that the static representation is not lost, and also in embodiments allows the model 116 to cope with missing prior images.

The text encoder 404 may also be denoted $E_{txt}(\cdot)$. It is arranged to receive the textual report 204 (e.g. from text database 114) and to encode this into a text embedding. The ML model 116 further comprises a loss function 420, such as a contrastive loss function, which is configured to compare the latent image representation $V$ with the text embedding by evaluating a suitable similarity metric, such as a contrastive loss metric. Over a plurality of training data points, each encoded and evaluated as described above, the method performed by the ML engine 118 is configured to train the ML model 116 by tuning the parameters (e.g. weights) of the image encoder 402, text encoder 404 and difference encoder (e.g. vision transformer) 412 so as to try to minimize the measure of loss between the latent image representation $V$ and the text encoding.

In embodiments, the text report 204 may be divided into one or more portions 414, e.g. sentences or clauses, before being input into the text encoder 404. The text encoder 404 may be configured to encode each such portion of the text 204 into a respective text-portion embedding, such that the text embedding for a given report 204 comprises a plurality of constituent text-portion embeddings corresponding to different portions (e.g. sentences or clauses) of the report 204. In such embodiments, then for a given data point (comprising a given current image 202_curr, prior image 202_prior and text report 204), the loss function 420 may be configured to evaluate the local similarity between each possible pairing of text embedding from the report 204 and image embedding from the corresponding current image 202_curr, and between each possible pairing of text embedding from the report 204 with individual patch embedding from the corresponding difference embedding $P^{diff}$ - i.e. to compare each text-portion embedding for the given data point with each present patch embedding for the same data point, and to compare each text-portion embedding for the data point with each patch difference embedding for the same data point. In other words for each text-portion embedding (e.g. sentence embedding), the local similarity is determined between the text-portion embedding and each of the elements of the combined image representation $V$. In this case the training comprises searching for an alignment between, on the one hand, the text-portion embeddings from the report 204, and on the other hand the elements of the combined image representation $V$ (which comprises the current patch embeddings from the current image 202_curr and the patch difference embeddings from the difference embedding matrix $P^{diff}$), whilst tuning the parameters (e.g. weights) of the image encoder 402, text encoder 404 and difference encoder 412 to try to minimize the measure of loss (maximize the similarity) between the text-portion embeddings and their best aligned patch embeddings from the combined image representation $V$. This way the model 116 will learn to be able to align portions of text in a report with features in a current image or differences between a current and prior image. Note that (at least in the described embodiments) the alignment is performed once using the joint representations. It is not necessary to not construct two separate affinity matrices, there is only one affinity matrix where the image embeddings $V$ encapsulate both $P^{diff}$ and current image content $P^{curr}$.

Alternatively or additionally, the loss function may compute a global similarity between the text of the report 204 and the combined image representation $V$ (which comprises the current image embedding $P^{curr}$ and a the difference embedding $P^{diff}$), and the training may be performed to try to maxims the global similarity (minimize the overall loss). In embodiments the training may be performed based on both the global similarity and local similarities.

Embodiments may employ classification text tokens, denoted CLS, to divide the text into portions 414. A CLS is a dedicated text token appended at the front of text inputs for text classification purposes. To form a global embedding in image analysis, a mean pooling may be performed which aggregates all the patch representations into a single vector. In text modelling, however, instead of performing mean pooling, a dedicated token called "CLS" may be used, which aggregates all the text token representations throughout the network. This way, the model can learn to filter-out content (a subset of tokens, e.g. not related to disease) and aggregate the information required for the task. It can be seen as smart or data-adaptive pooling. The CLS embedding is later used as global representation of the text data or report.

The ML model 116 may comprise a projection module arranged to perform a projection (i.e. normalization of the embeddings) such that the text embedding, current image embedding $P^{curr}$ and difference embedding $P^{diff}$ are normalized into a common latent space prior for comparison by the loss function 420. This may comprise normalizing the current image embedding and difference embedding so as to be the same size as the text embedding, or normalizing the text embedding so as to be the same size as the current image embedding and difference embedding, or normalizing al three to be the same size as one another. Alternatively projection may not be needed if the text encoder 402 and text encoder 404 are inherently configured to generate embeddings of the same size as one another, or if a similarity metric is used that does not rely on the embeddings from the image and text domains being the same size as one another.

Once trained, the ML model 116 may be deployed and used to make predictions. This may comprise receiving a query data point comprising a text query, a queried current image, and optionally a queried prior image showing the same target as the current image (e.g. same body part of the same patient) from an earlier point in time. The text query is input to the text encoder 404 to generate a text embedding. The text query may be divided into more than one portion (e.g. sentences or clauses) and an individual text-portion embedding generated for each portion. The queried current image is input to the image encoder 402 to generate a corresponding current image embedding comprising a plurality of patch embeddings corresponding to the different spatial subdivisions of the image space, as described previously for a training data point. If present, the prior image is also input to the image encoder 404 to generate a prior image embedding comprising a plurality of corresponding prior patch embeddings; and the present and prior image embeddings are input to the difference encoder 412 (e.g. into the vision transformer after spatial and temporal encoding) to generate a corresponding difference embedding comprising a plurality of constituent difference patch embeddings. The loss function 420 is then used to determine which patch embedding or embeddings from among the combined temporal image representation $V$ (comprising the current patch embeddings and difference patch embeddings) is/are most closely aligned with the text embedding of the text query, or each text-portion embedding of the query (i.e. most closely matches according to the similarity metric). This mapping may be output to the user 107 graphically through the UI console 106, such as by highlighting or outlining the region(s) in the current or prior image corresponding to the most closely aligned patch embedding(s) and linking them to the text or text portion(s) with which they are matched, e.g. by drawing a line between the region and the text portion or using a legend or colour coding to link the text and image region(s). For example this could be used to match propositions in text queries to features in medical scans to help determine the likely presence or absence of a disease, injury or other such condition.

Thus the trained model can be used to match text and image content, by relying on the fused imaged embeddings ($V$) and text embeddings. It compares the patch embeddings against query text embeddings in the learnt joint latent space by computing their similarities, e.g. cosine similarities, but unlike existing methods the image embeddings capture also the past information so the model enables more diverse queries, e.g. disease progression related text inputs.

Alternatively or additionally, the text query may comprise a draft report on the queried current image, and if the prior image is included in the query data point, optionally also reporting on a change compared to the prior image. In this case the prediction may comprise generating a proposed update based on the respective present image embedding and text embedding of the query, and if available also based on the corresponding difference embedding. The prediction can also work without a text query - i.e. the query data point may comprise only a current image, or only a current image and one or more prior images, but no draft report.

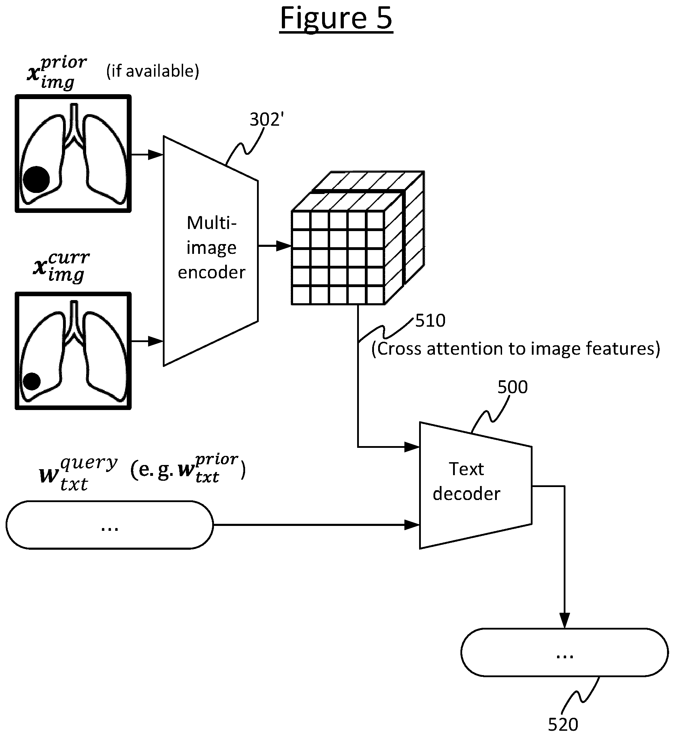

Figure 5 shows an extension to the model of Figure 3(c) and 4 for auto-generating reports or predicting updates to reports. The queried image(s) are input into the image encoder 302' (comprising the image encoder 402 and difference encoder 412) in order to generate an instance of the combined temporal image embedding $V$. The one or more queried images comprise at least a current image $x_{img}^{curr}$ and may also comprise, if available, one or more prior images $x_{img}^{curr}$. In response the image encode network 302' generates the corresponding embeddings $V$. If no prior image was available then the missing image embedding $P^{miss}$ is used in place of the difference embedding. A text query $w_{txt}^{query}$ is input to a text encoder 500, which may have been trained along with the encoders 402, 404, 412 during the pre-training. The text query may be divided into portions, e.g. sentences or clauses, such as by using CLS tokens or the like. In embodiments the text query $w_{txt}^{query}$ may comprise a draft report on the one or more queried images. Alternatively or additionally, the text query $w_{txt}^{query}$ may comprise a past report $w_{txt}^{prior}$ used as a prompt. The text decoder 500 is also arranged to receive a signal 510 from the image domain based on the combined temporal image embedding $V$, e.g. a cross-attention signal, such that the image domain can influence the decoding of the text. Based on the input text query $w_{txt}^{query}$ and the signal from the image domain 510, the text decoder 500 generates predicted text 520, e.g. based on causal self-attention to text features. The predicted text 520 may comprise a proposed update to the input text query, or an automatically generated report generated based on one or more prior reports $w_{txt}^{prior}$ as a prompt.

Alternatively no input text query need be present and instead the text decoder 500 may be operable to decode only the image domain embedding $V$ into the predicted text 520 (where $V$ again comprises the combination of current image embedding $P^{curr}$ based on the current image of the input query, along with either the difference embedding $P^{diff}$ if a prior image is available in the input query or the missing image embedding $P^{miss}$ if not). This enables an auto-generated report 520 to be generated based on an input query that comprises image data alone, and no text query such as a draft report or prior report. The input image data of such an input query may comprise either a current image $x_{img}^{curr}$ and prior image(s) $x_{img}^{prior}$, or just a current image $x_{img}^{curr}$ (in which case the learned missing image embedding is used in place of the difference embedding in the latent image space $V$). For example if it's the first visit of a patient (no past reports or images), the text decoder can still be utilised to decode a report.

The text query $w_{txt}^{query}$ (if present) is encoded within the model 500 (a constituent of model of the wider model 116) and decoded within that same model 500, referred to above as the text decoder 500. In embodiments the text decoder 500 and text encoder 404 are the same model, e.g. a CXR-BERT model. So the encoding of the text and the deciding of the text and images is all happening within a combined encoder/decoder model 500. In some such embodiments only a small-sized prediction head (e.g. multi-layer perceptron) need be added at the end of the encoder part of the model 500 in order to convert the high-dimensional text-token embeddings to token IDs. For instance, the final layer of BERT (404) outputs a high dimensional real-valued vector for each input token, then this prediction head converts those vectors into token ids. In the literature, it is known per se to include that prediction head as part of a BERT model (corresponding to either 500 or 404 in the present case).

Thus, as described in the preceding paragraphs, at deployment time (e.g. after fine-tuning), the text encoder 404, which was initially used to encode text inputs in pre-training, may be repurposed to perform both input encoding and text decoding. In more detail, the layers of the encoder/decoder model 404/500 process a set of input text embeddings (either prior or query) and also a set of patch embeddings, which are later decoded token-by-token to form an output report. This procedure is repeated iteratively until the final token is predicted. In this iterative procedure, each iteration decodes/predicts the next unknown token. The image embeddings drive the decoding process, the input text tokens are just used as queries to attend to the corresponding image patch embeddings ($V$). One can think about it as a big hash-table (image embeddings) and query text tokens (queries) attending to specific fields in this hash table to form an output representation.

In embodiments, if there is no text query to begin with, the model passes a specific token [BOS], beginning of sentence, that initiates the iterative decoding process. These models are also known as auto-regressive models. Previous model token predictions are passed in as inputs to predict the next unknown token. E.g. if the final output report contains 100 tokens, then the model is invoked 100 times sequentially to form this final output. However, the computation can be parallelised thanks to the casual attention between tokens. The decoding process is stopped once it hits the end of sentence token [EOS].

For instance, if a "[BOS]" (beginning of sentence) token is the first input, its final embedding at the output of 404 will be converted into a token id using a prediction head. Say that it is the token "[There]", then the first iteration is completed. In the next iteration, the model passes in "[BOS] [There]" and uses the embedding of token "[There]" to predict the next token, such "[is]". In the third iteration, it will be "[BOS] [There] [is]", and so on. And if there is any prior text or query, it is just prepended to the beginning of these tokens to set a context for the encoder and decoder model.

By whatever means generated, the generated text 520 (a proposed update or automatically generated report) may then be output as a suggestion to the user 107 through the UI console 106, which the user may choose to accept, reject or edit. E.g. if the user accepts the update, this is included in the report. A treatment to the target may then be actioned based on the report after having been reviewed by the ML model 116 in this manner. There are different ways in which the generated report or update may be presented to the user, e.g. I) upfront: the user reviews and edits the generated report; or II) after signing: the user generates their own report and then the machine presents the automatic report for comparison.

The image encoder 402 may also be fined-tuned after the initial training with a small number of manual labels. I.e. the disclosed approach can be generalised to encode also the prior reports during pre-training or fine-tuning to gain additional context at deployment time. This may be used for example, to detect abnormalities in the images and locate them, or to quantify progression of disease across scans or time points, or if the reporting radiologist has been monitoring the patient already over some time (e.g. days), then past reports can guide the model in terms of what to prioritise and what to look for in the current scans.

In some embodiments, the ML model 116 may further comprise a missing image encoder (not shown). In this case, at least one training or query data point may be missing a prior image 202_prior, such that it only comprises a current image 202_curr and a report 204. For example a patient may not always have a prior scan available. The missing image encoder is arranged to generate a missing image embedding $P^{miss}$ as a placeholder for the prior image embedding $P^{prior}$ that would otherwise be generated if the prior image was present. The missing image embedding is a trainable parameter that may be learned along with the training of the rest of the model 116, based on at least one training data point that is missing a prior image. I.e. the model learns a dedicated vector as a placeholder for the difference representations ($P^{diff}$). Intuitively, it informs that there is no image so no progression related content is available. The missing image embedding $P^{miss}$ is input to the difference encoder in lieu of the prior image embedding. In embodiments where the image embeddings output by the image encoder 402 comprise a plurality of constituent patch embeddings, the missing image embedding also comprises a corresponding plurality (e.g. grid) of embeddings but these are preferably all the same as since there is no information about the missing image then there is nothing to learn to distinguish between the missing patches (and models that are over-parametrized tend to be less good at making generalizations). However it is not excluded that the missing image embedding could comprise and independently learnable constituent embedding for each patch.

As another alternative or additional optional feature, the ML model 116 comprises a masked learning modelling (MLM) loss function 422 and an additional branch of the text encoding network. The masked language modelling may be applied in parallel with the contrastive loss based modelling for at least one of the training data points. This means, as well as the encoding of the text portions 414 and the determination of the similarity with the image and difference embeddings as described earlier, a copy 416 of the text of the respective report is also generated in which one or more parts of the text are masked (i.e. redacted). For example the text may be divided into tokens, where the tokens could be individual characters, groups of characters, syllables or words and one or more of the individual tokens may be masked in the masked text 416. The masked text 416 is then input into the text encoder 404 to encode it into a masked-text embedding. This encoding is also based on a cross-attention signal 418 from the image embeddings $V$. This may comprise encoding each of the non-masked tokens individually such that the masked-text embedding comprises a plurality of individual token embeddings, one for each token. A MLM module (not shown) of the ML model 116 generates a prediction for at least one of the masked parts, e.g. by predicting a token embedding for each of the masked tokens. The MLM loss function 422 then computes a MLM loss based on the masked-text embedding and prediction of the at least one masked part, and the training of the image encoder 402 and text encoder 404 is guided by the feedback signal.

To elaborate, some tokens are masked and some are unmasked (original input), and by utilising the image content and unmasked text data, we will try to guess the masked content. For instance, the masked text 416 may comprise: "the [MASKED] opacity has resolved since the last visit". To predict this, the model first needs to understand the context, it is set by the unmasked tokens, where it is required to look into the imaging data and search for opacities. Once the context is extracted, which is represented by text embeddings, then the model will perform cross-attention from these text embeddings to image patch embeddings to extract the key information we are looking for. E.g. say that the true answer is token: "left". By performing cross-attention from the text modality to image modality, it is possible to extract new text representations that correspond to token "left" and make the prediction based on that. The "cross" in "cross-attention" refers to cross modality. If the model makes a wrong guess for the masked token, e.g. "right" or "tomorrow", then it will penalise both the text and image encoders since either the context was wrong or image embeddings were not useful in answering the question at the time of cross-attention. The MLM provides an auxiliary training objective to provide an additional model supervision. Once the model is pre-trained, the MLM component may be discarded.

While the techniques disclosed above have been described in terms of each data point comprising a current image and one prior image, the proposed encoding strategy of multiple images is not limited to two scans only but can be linearly scaled to arbitrary number of past images, in embodiments via using cross-attention in the vision transformer block. Thus the described model and method could be extended to data points comprising more than one prior image by adding an additional branch to the image encoder network for each prior image per data point. The foregoing description and the figures have explained and visualised the method for the case of two images for the sake simplicity and particular applications may not require more than one prior image. However, if the reports describe a history going back further than one prior image, e.g. patient history covering a period for which more than one past scan is available, for example, then there would be value in processing more than two scans.

Figure 2B shows a method that may be performed using the machine learning model 116 once trained - in the so-called "deployment" stage or "in-the field", i.e. when actually being used for an application rather than just being trained. Note that describing the model 116 as trained, or being used in the deployment stage or such like, does not exclude that further training could be performed. Indeed, in embodiments the model 116 can keep learning in an ongoing fashion during the deployment stage as well. To say that the ML model 116 has been trained implies only that a certain amount of training has been performed in order to allow it to be used with some reasonable degree of performance.

At step R10, an input image is received into the input of the image encoder 402, and at step R20 an input text query is input into the input of the text encoder 404. The input image is another instance of a current image 202_curr that may be input to the text image model 402 as shown in Figure 4, but this time at the deployment stage rather than a training stage. Similarly the text query is another instance of text 204 that may be input into the text model 403 as shown in Figure 4, but again now at deployment rather than training. A corresponding prior image 202_prior may also be input to the image encoder 402.

The input image(s) 202 may be selected by a user via a user interface (UI) of the UI console 106. It may be retrieved from the image database 112 or captured from the image source 102, e.g. a scanner or camera. The input image 202 input at the deployment stage may comprise an image of anything that the user 107 wishes to analyse using vision-text processing. For example the input image 202 may comprise a 2D or 3D bodily scan of a human or animal, or a body party of the human or animal, obtained from the scanner 102. Or the image 202 could comprise an image or a physical object or substance which the user 107 wishes to analyse; e.g. an image of an electronic, electrical or mechanical device (or part thereof) which the user wishes to perform diagnostics on; or a physical object (or part thereof) or substance, such as a manufactured object or substance, which the user 107 wishes to assess the state of, e.g. for quality control purposes.

The text query may be entered by the user 107 through the UI of the UI console 107, or may be selected by the user 107 through the UI of the UI console to be retrieved from the text database 114. The text query - which could also be called a text prompt or just input text - may comprise any portion of text 204 input at the deployment stage which comprises one or more propositions about a possible condition or state of the being, object or substance (or part thereof) shown in the corresponding input image 202. For instance, the input text query 204 may comprise a proposition about the occurrence or absence of a possible condition, e.g. a medical condition such as a disease or illness, of a bodily part of a human or animal shown in the image 204. Or the input text query could comprise a proposition about the possible occurrence or absence of a certain condition of an object or substance (or part thereof) shown in the image, e.g. a state of wear or deterioration of the object or substance, or a quality of the object or substance, or a possible fault in the case of an electronic, electrical or mechanical device. As yet another possibility, in an image recognition scenario such for presence detection or object detection, the input text query 204 may comprise a proposition about the possible presence or absence of a member of a particular species (e.g. human) or a particular body part (e.g. human face), or the presence or absence of a particular individual (e.g. particular person or particular person's face), or the presence or absence of a particular type of object (e.g. a vehicle) or a particular instance of an object (e.g. vehicle with a particular license plate). The input text query could be any length, whether one or more sentences, one or more paragraphs, one or more sections of a document, a whole document, or more than one document. Note also that "query" in this context does not necessarily imply that the text in question is phrased grammatically speaking as a question. Rather it could be any proposition phrased as a statement, question or otherwise.

At step R30 the image encoder 402 generates the patch embeddings $V$, for the input image(s) 202_curr, 202_prior. At step R40 the text encoder 404 generates at least one text embedding from the input text query 204. In embodiments the input query may be broken down into more than one portion, such as separate sentences, clauses, paragraphs or sections, and the text encoder 404 may generate an individual text embedding for each such.

Note: steps R10 and R20 could be performed in either order with respect to one another or in parallel. Similarly steps R30 and R40 could be performed in either order with respect to one another or in parallel. In other words it doesn't matter whether the images or text are processed first, or in embodiments they could be processed at least partially in parallel.

At step R50, the ML engine 118 performs a comparison between the patch embeddings V from the image domain and the text embedding or one or more text-portion embeddings. The ML engine 118 searches for regions of the image space where the present image or difference embedding $P^{dif}$ where the text embedding or a text-portion embedding is above a threshold similarity with the local patch embedding. This process may be referred to as local alignment or "grounding". The idea is to find a specific region or regions within the image space that can be linked to a proposition in the text query.

At step R60, the ML engine 118 outputs data which can be used to control the UI on the UI console 106 to render an indication of the connection between the text query 204 and the linked region in the present or past image 202_curr, 202_prior, or between each portion of the text query (e.g. each sentence) and the linked regions. For example the UI may display the image on screen, and highlight the region or regions in question, or drawing an outline around the region or regions. The text query may also be shown on screen and could be linked to the aligned region or regions e.g. by suitable colour coding, or a graphical or alphanumeric legend mapping the aligned region(s) to the text, or by overlaying the text on the aligned region(s), or linking it by a line rendered between the text and the aligned region(s), etc.

Figure 2C shows an example of a particular method that may be implemented using the system of Figure 1 and model of Figure 4. The method is to be used to estimate a condition (e.g. disease or cause of death) of a target subject. The target subject is either a human or an animal (e.g. mammal, bird, fish, marsupial or invertebrate).

Prior to the method there is a stage of training the machine learning model 116 as discussed previously. The model 116 is trained based on a plurality of training data points (preferably tens, hundreds, thousands or more) captured from one or more subjects, together with associated reports including text authored based on a review of the respective scans. The past scans preferably comprise one or more of scans at least one subject other than the target subject (but of the same species). They may also include one or more past scans of the target subject. The reports of the past scans may have been authored purely manually, or with AI assistance. They may have been authored by the same user 107 who is to author the new report on the target subject using the method of Figure 2C, or a different user, or a combination. Preferably the past reports include at least some reports authored by different users so as to introduce some variation in style into the training data. Either way, the training may comprise a supervised approach, reinforcement approach or unsupervised approach.

The machine learning model 116 is thus trained to be able to receive the image(s) of a current scan and an associated report, and to output suggestions for updating the text of the report.

At step S10) at least one current scan 202_curr is taken of the target subject using the at least one scanner 102. As discussed earlier this may comprise for example an x-ray based scan (e.g. CT scan), a PET scan, an MRI scan or an ultrasound scan, or a combination. Each scan 202 may comprise a single 2D image, or a 3D stack of 2D images (e.g. as set of "slices" through the subject). A prior scan 202_prior of the same target may also be retrieved from the image database 112 or elsewhere.

At step S20), the user (e.g. radiologist, vet or researcher) 107 reviews the current scan 202_curr, and prior scan 202_prior if available, though the user interface (UI) displayed on the UI console 106.

At step S30), based on the review, the user 107 authors a draft report 204 (or at least part thereof) - at this stage using his/her professional judgement only (not AI). The report 204 comprises text written by the user 107. The text comprises a description of one or more possible conditions (e.g. diseases) which the target subject may have based on the review. The text may also include an explanation of why the user 107 came to this conclusion, e.g. what features in the image(s) led to this.

At step S40) the image data of the scan(s) 202_curr, 202_prior are input into the machine learning model 116, together with at least the text of the corresponding report 204. Based on the report and image(s) together, the machine learning model 116 generates one or more suggestions for amendments 206 to the text of the report 204. For example the suggestions may comprise the addition of one or more potentially missing details, the correction of one or more possible errors, and/or one or more suggestions for alternative terminology (e.g. for clarity or to promote consistency across reports).

At step S50) the suggested amendments 206 are presented to the user through the UI on the UI console 106. For example they may be presented on screen in the form of a proposed updated version of the report, e.g. with the amendments shown tracked (marked-up) on the previous version of the report (such as by means of strikethrough, underlining and/or highlighting). Further the machine learning model 116 is able to identify which part of the image (or which part of which image) resulted in each suggestion. This is indicated visually to the user 107 through the UI, linking each suggestion to a particular corresponding feature in the image (or one of the images). In other words the UI indicates to the user the cause of each suggestion in the image. Some examples for this will be discussed in more detail shortly with respect to Figure 3B.

The user 107 then reviews each of the one or more suggestions made by the machine learning model 116. However the suggested amendments are not finalized autonomously by the AI. Only if the user 107 deems fit based on his/her professional judgement will he/she choose to accept each update to be included into an actual updated version of the report. The user 107 may accept the suggestions though the UI, for example by selecting to accept or reject each one individually, or selecting accept all or reject all. The selection is made through the UI on the at least one UI console 106, e.g. via a mouse, touchscreen, keyboard or voice input. As another possibility, upon being prompted to reconsider the wording by the suggestion from the machine learning model 116, the user 107 may choose to make an alternative amendment that was neither the original wording nor the suggestion from the ML model 116.

If the user 107 accepts one or more of the suggested amendments, and/or makes one or more amendments of his/her own, then optionally steps S30) to S50) may be repeated with the updated report in order to generate one or more updated suggestions. That is, the amended report (as amended in the first iteration of the method) is input into the machine learning model 116, again along with the corresponding image data of the one or more current scans. The machine learning model 116 processes these together in order to output one or more updated suggestions, which are presented to the user 107, and which the user may again accept or reject, or may make one or more further alternative amendments of his/her own, thus creating a further updated version of the report. In embodiments, the method may continue over one or more yet further iterations in a similar manner, each time the ML model 116 analysing the previously updated version of the report.

Note the method shown in Figure 2C may be somewhat schematized compared to the actual implementation. For instance, in embodiments it is not necessary for the user 107 to compose the entire report 204 and then input it as a whole into the model 116 before the suggestions 206 can start to be generated. In embodiments, the suggestions could be generated and output to the user 107 live as he or she types the report. I.e. the suggestions are output dynamically, or "on the fly", in real-time as the user types, with one or more suggestions being generated and output to the user 107 based on the report as composed so-far before the user 107 has finished writing the whole report.

In embodiments the training of the machine learning model 116 may also be refined with each iteration of the method, i.e. based on the user accepting or rejecting the model's suggestions in each of the one or more rounds of the method.

In some scenarios, then at some point after at least one iteration of the method, one or more additional scans of the target subject may be performed. This adds new image data into the system as well as updated report data. For example, a new scan of the target subject may be performed after a matter of hours, or the next day, next week, next year or next year. The arrow labelled "time" in Figure 2C represents the possibility of additional, future scans being added over time (where the different schematized icons labelled 202 in the figure represent different scans taken on different occasions). This provides one or more new images of the target subject, which may be added only after the initial report has been authored and reviewed at least once by the ML model 116. In this case, the method may comprise inputting the new image data from the one or more additional scans into the machine learning model 116, along with the latest version of the report (including any amendments accepted by the user 107). Based on these, the ML model 116 may the output one or more additional suggestions taking into account the new images. These are output to the user 107 through the UI, in order for the user to review and accept or reject them based on his/her judgement, in a similar manner as described earlier.

In embodiments, the training of the machine learning model 116 may also be refined based on the image(s) of the additional scan(s), and optionally any additional amendments which the user 107 accepts, rejects or makes him/herself based on the new scan(s).

It will be appreciated that Figure 2C is just one example workflow. Other methods involving the alignment of text queries with image regions may also be implemented using the ML model disclosed herein.

## EXAMPLE IMPLEMENTATION

The following now describes an example implementation of the above described techniques, in an example application of biomedical vision-language processing.

Self-supervised learning in vision-language processing (VLP) exploits semantic alignment between imaging and text modalities. Prior work in biomedical VLP has mostly relied on the alignment of single image and report pairs even though clinical notes commonly refer to prior images. This does not only introduce poor alignment between the modalities but also a missed opportunity to exploit rich self-supervision through existing temporal content in the data. In the present disclosure, we explicitly account for prior images and reports when available during both training and optionally fine-tuning. Embodiments of the disclosed approach may be named herein BioViL-T, which uses a CNN-Transformer hybrid multi-image encoder trained jointly with a text model. It is designed to be versatile to arising challenges such as pose variations and missing input images across time. The resulting model excels on downstream tasks both in single- and multi-image setups, achieving state-of-the-art (SOTA) performance on (i) progression classification, (ii) phrase grounding, and (iii) report generation, whilst offering consistent improvements on disease classification and sentence-similarity tasks. Our experimental results show the significant advantages of incorporating prior images and reports to make most use of the data.

Self-supervision from image-text pairs has enabled the development of flexible general-purpose vision-language models both in the general domain and for specialised domains such as biomedicine and radiology. Vision-language processing (VLP) has shown that cross-modal supervision can provide a richer signal for training both image and text models. However, the success of VLP relies on paired samples sharing semantics, i.e. given an image and text pair, the text should preferably describe the image with minimal extraneous detail.

In this regard, VLP in biomedicine and radiology poses a distinctive challenge, as reports routinely include comparisons to prior imaging studies. Without knowledge of this prior image, temporal information in the text modality, e.g. "Pneumonia is improving", could pertain to any image containing "Pneumonia", producing ambiguity during contrastive training (Figure 1). Despite this, the existing VLP work to date considers alignment between only single images and reports, going so far as to remove temporal content from reports in training data to prevent 'hallucinations' in downstream report generation. However, temporal information can provide complementary self-supervision, solely by exploiting existing structure, and without requiring any additional data.

In the present work, we neither ignore nor remove temporal information in the text modality, but explicitly account for it during pre-training. Rather than treating all image-report pairs in the dataset as independent, we exploit temporal correlations by making prior images available for comparison to a given report. To learn from this structure, we develop a temporal VLP pre-training framework named BioViL-T. A core component is its new multi-image encoder that can handle the absence of prior images and potential spatial misalignment between images across time. BioViL-T takes into account prior images where available, removing cross-modal ambiguity as illustrated in Figure 3. Linking multiple images during pre-training proves beneficial to both image and text models: we report state-of-the-art (SOTA) performance on both temporal image classification and report generation. In the latter case, we show that prefixing the prior report substantially increases performance, again reflecting the value of prior information. We emphasise that the benefit is not restricted to temporal downstream tasks: our approach also achieves SOTA on non-temporal tasks of pneumonia detection and phrase grounding, underscoring the value of a cleaner learning signal during VLP without needing to modify or add to the training dataset. Embodiments may provide one, more or all of the following contributions.

- We introduce a novel pre-training framework, called BioViL-T, which leverages the temporal relationship of samples to self-supervise VLP models. BioViL-T extends the applicability of biomedical pre-trained models to a new set of downstream tasks without compromising performance on existing benchmarks.
- We develop a generic multi-image encoder that handles missing image inputs and incorporates longitudinal information without requiring explicit image registration.
- We achieve SOTA results in biomedical report generation, temporal image classification, and phrase grounding downstream benchmarks by accounting for prior context in self-supervised training and fine-tuning.

Figure 3 illustrates the following. (a) Existing visual-language pre-training approaches often use only a single image for contrastive learning (e.g. InfoNCE ). (b) In such settings, discarding the temporal connectivity of images limits the alignment of image-text pairs as shown with the affinity matrix, leading to suboptimal pre-training and missed opportunity to create additional model supervision for free. (c, d) Our approach exploits this domain knowledge by learning to incorporate a series of images and correlate them to reports, leading to pre-trained models that can generalise to a wider range of downstream tasks whilst achieving SOTA performance.

The disclosed approach comprises a multi-image encoder designed to extract spatio-temporal features from sequences of images and a text encoder incorporating optional cross-attention on image features. The models are trained jointly with image-guided MLM and cross-modal global and local contrastive objectives. The resulting image and text models are later adapted for uni- or multimodal downstream tasks.

For a given image and report pair ( $x_{img}^{curr}$ , $x_{txt}^{curr}$ ), the report $x_{txt}^{curr}$ describes the current image content and changes in reference to prior images. The below formulation focuses on a single prior image; however, it can be generalised to *multiple* prior images depending on the application. Hence, we construct datasets by including the prior image whenever it exists: $(x_{img}^{curr}, x_{img}^{prior}, x_{txtimg}^{curr}) \in D_m$ or $(x_{img}^{curr}, \emptyset, x_{txt}^{curr}) \in D_s$ with the resulting dataset being a union of single and multi-image examples: $D = D_m \cup D_s$.

Figure 4 illustrates the disclosed self-supervised VLP training framework BioViL-T: Image representations *V* are extracted from single and multiple input scans (whenever available) using a hybrid CNN and transformer encoder. This design choice is to increase the data efficiency and enable the fusion of temporal content without requiring image registration. They are later matched with their corresponding text representations obtained with CXR-BERT using local and global InfoNCE training objectives. As an additional model supervision, multi-modal fused representations, obtained with cross-attention, may be used for image-guided masked language modelling.

**Extracting spatio-temporal image features**

Clinical findings are often observed across different image regions and co-occur simultaneously, which requires dense level visual reasoning across time to capture both static and temporal features. In contrast to late global fusion and bounding-box based approaches, BioViL-T leverages local correspondences between image regions across time using transformer self-attention blocks. Thus our method does not require an explicit image registration step between time points.

We disclose a hybrid CNN-Transformer encoder model due to its data efficiency and spatial flexibility of cross-attention across time points: $E_{img} : \mathbb{R}^{W \times H} \to \mathbb{R}^{W \times H \times D_{img}}$ and $A_{img} : \mathbb{R}^{T \times L \times D_{img}} \to \mathbb{R}^{L \times D_{img}}$, where $W$, $H$, and $T$ correspond to spatiotemporal dimensions, $L = W'H'$ is the number of visual tokens per image, and $D_{img}$ is the embedding dimension. Here $E_{img}$ (e.g. ResNet-50) serves as a stem network to provide visual token features of individual images. The CNN's inductive biases ensure data efficiency of our hybrid model, making it ideal for smaller scale biomedical datasets. $E_{img}$ is initialised with BioViL weights. The main purpose of $A_{img}$ is to capture patch embedding interactions across time when a prior image $x_{img}^{prior}$ is available and to aggregate them into a fixed-length token representation.

Input visual tokens, $H_0^{curr} = \mathrm{P}^{curr} := E_{img}(x_{img}^{curr}), H_0^{prior} := E_{img}(x_{img}^{prior})$ are augmented with spatial and temporal positional encodings and flattened across the spatial dimensions. They are then processed by $K$ transformer encoder layers A as follows:

$$
\begin{bmatrix} H_k^{curr} \\ H_k^{prior} \end{bmatrix} = A_k \left( \begin{bmatrix} H_{k-1}^{curr} + S + 1_L \otimes t^{curr} \\ H_{k-1}^{prior} + S + 1_L \otimes t^{prior} \end{bmatrix} \right), \tag{1}
$$

for $k = 1,...,K$, where $S \in \mathrm{R}^{L \times D_{img}}$ denotes 2D sinusoidal positional encodings and $T = [t^{curr}; t^{prior}] \in \mathrm{R}^{2 \times D_{img}}$ is its temporal counterpart, which is learnt (Fig. 4). The layer-normalised (LN) output of the final transformer encoder block $\mathbf{P}^{diff} := \mathrm{LN}(\mathbf{H}_K^{curr})$ is an 'aggregated' representation of patch-level progression information anchored on the current image. Figure 4 shows attention roll-out applied to $\mathbf{P}^{diff}$ after pre-training, showing how the prior image contributes to the fused representation. The encoder is robust against variations in pose such that registration is not necessary.

**Static-temporal feature decomposition:** When a prior image is available the final image representation $V := \mathrm{P}^{curr} \bigoplus \mathrm{P}^{diff} \in \mathbb{R}^{W \times H \times 2D_{img}}$ is formed by concatenating two sets of features: those from the current image alone ($\mathbf{P}^{curr}$) and the temporal features from current and prior images ($\mathbf{P}^{diff}$). In this way, self-attention is mainly required to cope with pose variations and patch comparisons across time in extracting temporal content, removing the need for registration or explicit spatial feature alignment. When no prior scan is available (x E Ds), $A_{img}$ is not used and $\mathbf{P}^{diff}$ is replaced by a learnable token $\mathrm{P}^{miss} \in \mathbb{R}^{D_{img}}$, replicated across the spatial dimensions. $A_{img}$ highlights the value of feature decomposition for tasks such as phrase grounding which require well-localised features.

Hereafter, downstream tasks that require solely single image features, $\mathrm{P}^{curr}$, are referred to as *static tasks,* and the ones that benefit from additional progression information, $\mathrm{P}^{diff}$, as *temporal tasks,* e.g. report decoding.

**Text-supervision for spatio-temporal learning**

Let $\mathbf{w} = (w_1,..., w_M)$ denote a vector of M tokens of a report $\mathbf{x}_{txt}$ after tokenisation. We first obtain contextualised token features $E_{txt}(\mathbf{w}) \in \mathbb{R}^{M \times D_{txt}}$ by passing a sequence of text tokens $\mathbf{w} = (w_1,..., w_M)$ through a BERT encoder $E_{txt}$. The input sequence is prepended with either a [CLS] or [MLM] token associated with a downstream training objective, conditioning the output features similar to. During training, we do two forward passes through $E_{txt}$: once with masking

at 45% probability (for the MLM objective) and once without masking for contrastive learning, as shown in Figure 4. The text encoder is initialised with the weights of CXR-BERT, a model pre-trained on domain-specific vocabulary and corpora.

Both text and image features are later projected into a joint latent space with $\phi_{\text{txt}}$ : $\mathbb{R}^{D\text{txt}} \to \mathbb{R}^{D}$, and similarly

$$v_{w,h}^{proj} = \phi_{img}(v_{w,h})$$ where $\phi_{\text{img}}$ : $\mathbb{R}^{D\text{img}} \to \mathbb{R}^{D}$, with $\phi$ being a two-layer perceptron in our experiments.

**Contrastive objectives:** Let $\mathbf{r} := [E_{\text{txt}}(\mathbf{w})]_{\text{[CLS]}}$ denote the global representation of w, with $\mathbf{r}^{\text{proj}} := \phi_{\text{txt}}(\mathbf{r})$ its projected version. Given projected patch embeddings $v_{w,h}^{proj}$, we can compute a global cosine similarity $S_c(\overline{v}^{proj}, r^{proj})$ and a local similarity using weighted pairwise cosine similarities across text tokens and projected patch embeddings. These similarities are used in both global and local contrastive objectives with the InfoNCE loss. The local loss proves crucial both for static phrase-grounding and temporal image classification (see Table 4), highlighting the importance of localised self-supervision.

**Image-guided masked language modelling:** Prior work has shown that biomedical visual-language learning benefits from an auxiliary task such as MLM since capturing the joint distribution of tokens can stabilise and improve language understanding during joint learning. Given a batch $\mathcal{B}$ of token vectors **w**, it is often defined as the cross-entropy for predicting the randomly sampled masked tokens, $m \subset \{1,...,M\}$, LMLM

$$= -\frac{1}{|\mathcal{B}|} \sum_{\mathbf{w} \in \mathcal{B}} \log p_\theta(\mathbf{w}_m \mid \mathbf{w}_{\backslash m}),$$

where $\vartheta$ are the weights of the text encoder $E_{\text{txt}}$.

In the absence of image information, however, certain masked findings and attributes are not readily predicted, e.g., "[MASK] is worsening". As shown in the general domain, visual information can help disambiguate such masked predictions and provide additional cross-modal supervision. Thus, we use cross-attention to the image features $v_{w,h}^{proj}$ during this task. Specifically, for our imageguided MLM objective we model $p_\theta(\mathbf{w}_m \mid \mathbf{w}_{/m}, \mathbf{v}_{w,h}^{\text{proj}})$.

**Adaptations to downstream tasks**

BioViL-T can be adapted to various downstream tasks. For phrase-grounding and zero-shot inference, we use $Sc(\mathbf{r}^{\text{proj}}, \mathbf{v}_{w,h}^{\text{proj}})$. For multiple-text prompts, projected text embeddings are marginalised prior to $\ell_2$-normalisation. To enable language decoding, $v_{w,h}^{proj}$ inputs are cross-attended by text queries w, and causal-attention is utilised between text tokens. Report generation tasks can greatly benefit from temporal joint latent space.

**Conditioning on prior reports:** In contrast to existing work, we incorporate the prior report as a prompt to contextualise the report generation task: $p_\Phi\left(w_{txt}^{curr} \mid w_{txt}^{prior}, v_{h,w}^{proj}\right)$, where $\Phi$ are the multi-modal encoder-decoder network's weights, and $w_{txt}^{curr}, w_{txt}^{prior}$ denote text tokens for current and prior reports respectively. This is analogous to finetuning GPT-3 with prompts and instructions, but conditioning on both images and the previous report. A dedicated separation token [SEP] is added into the input sequence $[w_{txt}^{prior}, \text{[SEP]}, w_{txt}^{curr}]$.

**Curation of imaging datasets:** CXR datasets often contain multiple image acquisitions $Z = \{x_1^{\text{img}}, ..., x_Z^{\text{img}}\}$ in a single visit due to data quality issues such as a limited field-of-view or scanning the wrong body part. We conduct curation to choose higher quality images among the potential candidates instead of performing a random selection. For this step, a separate BioViL-T is trained on 'clean' studies with single acquisitions and later used in a zero-shot setting to detect out-of-distribution samples arising from the reimaging process. The candidate $\hat{z}$ is selected as follows: $\hat{z} =$

$$argmax_{z \in Z} S_C\left(\overline{v}_z^{proj}, r^{proj}\right) s.t. \left|s_{\hat{z}} - s_{Z \setminus \hat{z}}\right| > \delta$$ for a margin $\delta$. This approach is applied to enhance the quality of the temporal classification dataset given its limited size.

## EXPERIMENTS

Here, we demonstrate BioViL-T's data efficiency and adaptability to a wide range of applications, and show how the model achieves SOTA performance on various downstream tasks by learning from data instances linked across time, making effective use of domain priors and the available training data. Specifically, our model is evaluated on a diverse set of downstream tasks including zero- and fewshot static and temporal image classification, language decoding (report generation), phrase-grounding, and sentence similarity.

**CXR-T benchmark:** We use a multi-modal benchmark dataset, *CXR-T,* to evaluate biomedical VLP models on two distinct temporal tasks: image classification and sentence similarity. The former comprises multi-image and ground-truth label pairs ($N$ = 1326) across 5 findings, with classes corresponding to 3 states of disease progression for each finding: {Improving, Stable, Worsening}. The latter quantifies the temporal-semantic similarity of text embeddings extracted from pairs of sentences ($N$ = 361). The pairs can be either paraphrases or contradictions in terms of disease progression. The data for both tasks was manually annotated and reviewed by a board certified radiologist.

**Datasets:** For pre-training, we use the MIMIC-CXR v2 chest X-ray dataset, which contains longitudinal imaging studies with corresponding radiological reports. We only use frontal view (AP/PA) scans and discard samples where reports do not contain an IMPRESSION section. From this data, we gather 174.1k and 4.9k text-image pairs for model training and validation respectively, with a majority of text-image pairs including a prior image: $\left|\mathcal{D}_m^{train}\right| = 118.8k, \left|\mathcal{D}_s^{train}\right| = 55.3k$. The text consists of the IMPRESSION section and, for MLM additionally the FINDINGS section if available. Note that *no manual labels* are used during pre-training and *no additional data* is used for the methods that leverage the link between current and prior images. For early stopping we track the validation loss.

Downstream evaluations are performed on a disjoint held-out test set shared across all tasks, $|D^{test}|$ = 2971. For report generation, we extend this test set with samples from healthy subjects (N = 815) to match the prevalence of pathological studies used in prior work. For fine-tuning on temporal image classification, we use labels from the Chest ImaGenome dataset. In detail, we use the following benchmark datasets: (I) MS-CXR for phrase grounding, (II) the RSNA Pneumonia dataset to test zero-shot and fine-tuned classification, (III) *CXR-T* for temporal sentence similarity and temporal image classification.

**Comparison approaches:** We compare our approach to other domain-specific SOTA pre-training frameworks specifically on phrase-grounding and zero-shot predictive performance. The non-temporal BioViL framework is most similar to our approach and provides insight into non-temporal pre-training. Our ResNet and BERT models are initialised with BioViL weights and architecturally have only added the transformer encoder block to support multiple images, and cross-attention for image-guided MLM. We additionally compare to internal ablations such as re- moving the past report during report generation and masking prior images during phrase grounding. For SOTA performance comparison, various AR and nearest-neighbour (NN) based language decoding approaches are used as baselines: IFCC, R2Gen, CXR-RePaiR-2, and CXR-RePaiR-Select.

For the temporal classification task, we compare against a baseline exploiting the BioViL image encoder, and an approach that makes use of graph convolutions across regions of interest extracted from bounding boxes. For BioViL, we perform affine image registration (with 4 DoF) for each pair of scans to cope with pose variations, and the encoded images are concatenated along the feature dimension and classified via a multilayer perceptron. We compare to the three-class setting. Lastly, we benchmark our final text model in isolation against domain specific SOTA models in a temporal sentence similarity task: CXR-BERT and PubMedBert.

For metrics due to class imbalance, we report macroaccuracy for temporal image classification. For phrase grounding, we use mean Intersection-Over-Union (mIoU) and Contrast-to-Noise-Ratio (CNR). The latter measures the discrepancies between cosine similarities inside and out of the bounding box region without requiring hard thresholds. To evaluate the quality of generated reports, we use both the standard lexical metrics, e.g., BLEU, ROUGE-L, and also domain-specific factuality metric: CheXbert. To directly probe the generation of change related information, we introduce a new metric called temporal entity matching (TEM) to compute the match score of a fixed set of temporal entities

## Results

| | Method | Pre-training | PI / PR | BLEU-4 | ROUGE | CHEXBERT | TEM |
|---|---|---|---|---|---|---|---|
| NN | CXR-RePaiR-2 | BioViL | ✗ / ✗ | 2.1 | 14.3 | 28.1 | 12.5 |
| | Baseline (NN) | BioViL | ✗ / ✗ | 3.7 | 20.0 | 28.3 | 11.1 |
| | Proposed (NN) | BioViL-T | ✓ / ✗ | 4.5 | 20.5 | 29.0 | 13.0 |
| AR | Baseline (AR) | BioViL | ✗ / ✗ | 7.5 ± 0.1 | 27.9 ± 0.1 | 29.3 ± 0.3 | 13.8 ± 0.1 |
| | Proposed | BioViL-T | ✓ / ✗ | 8.2 ± 0.1 | 28.7 ± 0.1 | 30.2 ± 0.7 | 16.0 ± 0.3 |
| | Proposed | BioViL-T | ✓ / ✓ | **9.2 ± 0.3** | **29.6 ± 0.1** | **31.7 ± 1.0** | **17.5 ± 0.1** |

Table 1. Results for report generation task: Predictions are evaluated in terms of lexical (BLEU-4, ROUGE) and factuality metrics (CHEXBERT, TEM). Approaches are grouped into two broad categories: nearest-neighbour (NN) and auto-regressive (AR). BioViL-T pre-training consistently yields improved decoding. Further, the consistent performance gains of using prior image and report demonstrate the importance of such domain priors. 'PI / PR' indicate usage of prior image and report, respectively.

| | Method (% of labels) | Pre-train | Consolidation | Pl. effusion | Pneumonia | Pneumothorax | Edema |
|---|---|---|---|---|---|---|---|
| Z&F | BioViL-T prompt (0%) | Temporal | 49.9 ± 3.1 | 59.8 ± 0.7 | 54.1 ± 3.0 | 37.9 ± 3.9 | 65.3 ± 1.4 |
| | BioViL-T (10%) | Temporal | 59.7 ± 2.4 | 62.4 ± 1.4 | 60.1 ± 2.1 | 35.3 ± 2.6 | 62.6 ± 1.7 |
| Supervised | CNN + Transformer | ImageNet | 44.0 ± 2.0 | 61.3 ± 1.6 | 45.1 ± 3.5 | 31.5 ± 3.1 | 65.5 ± 1.1 |
| | CheXRelNet | ImageNet | 47 | 47 | 47 | 36 | 49 |
| | BioViL | Static | 56.1 ± 1.5 | 62.3 ± 1.1 | 59.4 ± 1.0 | 41.7 ± 2.8 | 67.5 ± 0.8 |
| | BioViL w/reg | Static | 56.0 ± 1.5 | 63.0 ± 0.9 | 60.2 ± 0.7 | 42.5 ± 2.7 | 67.5 ± 0.9 |
| | BioViL-T | Temporal | **61.1 ± 2.4** | **67.0 ± 0.8** | **61.9 ± 1.9** | 42.6 ± 1.6 | **68.5 ± 0.8** |

Table 2. Temporal image classification results (repeated for 4 random seeds) on the *CXR-T* benchmark for fully-supervised and zero-/few-shot (Z&F) learning settings, in terms of macroaccuracy across the three classes for each finding. Affine registration is performed for the baseline method (denoted with suffix 'w/reg'), to partially address the pose variations across scans.

Table 3. Report generation results obtained with SOTA baseline methods using the same train/test splits. For comparison, we report the same lexical (BLEU-2) and factuality (CHEXBERT) metrics, and the specific sections decoded by each method.

| Method | Decoded sections | BLEU-2 | CHEXBERT |
|---|---|---|---|
| R2gen | Findings & Impression | 21.20 ± 0.10 | 14.80 ± 0.30 |
| IFCC | Findings | 21.70 ± 0.10 | 27.00 ± 0.40 |
| CXR-RePaiR-Sel. | Impression | 5.00 ± 0.10 | 27.40 ± 0.30 |
| BioViL-T | Impression | 15.86 ± 0.14 | 34.83 ± 0.73 |
| BioViL-T | Findings & Impression | 21.31 ± 0.19 | **35.86 ± 0.35** |

**Temporal pre-training yields data efficiency:** downstream tasks are enabled with minimal labels.

The sections 'NN' and 'Z&F' on Tables 1 and 2 report zero- and few-shot performance on tasks benefitting from temporal information: temporal image classification and report generation. Here we measure the quality of the learnt joint latent space and the extent to which BioViL-T enables efficient use of raw data. For zero-shot classification we prompt the AR fine-tuned model with prefix: "[FINDING] is" and compare the next-token probability of words meaning 'improving', 'stable', and 'worsening'.

Without using any labelled data, Table 2 shows that the proposed AR-based approach already yields performance superior to prior fully-supervised work on temporal image classification. With only 10% of labels, classification fine-tuning provides a further boost, indicating that BioViL-T produces a multi-image encoder readily adapted to temporal tasks. Similarly, in a zero-shot report-retrieval setting, the findings show that compared to temporally agnostic pre-training, BioViL-T leveraging prior images improves across all metrics. Consistent with prior work, the retrieved reports already preserve factuality with high CheXbert scores, more-so than the other metrics which measure fine-grained specifics of phrasing. This demonstrates that the latent space captures the high-level semantics of the clinical features. Fine-grained phrasing however will be substantially improved by AR fine-tuning.

**II. Achieving SOTA performance with BioViL-T:** A wide range of downstream tasks benefit substantially from temporally-aware pre-training.

Through downstream adaptations and fine-tuning our model, we report SOTA performance on report generation and temporal image classification tasks. For the former, using both prior images *and* reports during fine-tuning substantially improves across metrics (Table 1). In particular, TEM metric results show that temporal context is key for accurately describing change in the generated report while avoiding hallucinations. Comparing to published results on a comparable test split and metrics, we conclude that BioViL-T with finetuning achieves SOTA on report generation, producing reports that are lexically on par with prior work but substantially more factually accurate. Note that we do 'vanilla' AR fine-tuning to focus on the impact of the pre-trained encoders, so application-specific supervision could be used in conjunction to further boost performance.

In temporal image classification (Table 2), BioViL-T pretraining outperforms the non-temporal baseline (BioViL) and improves on previously-reported results by up to 20 percentage points (pp). Furthermore, baseline methods that rely on image registration (BioViL w/reg), underperform compared to the proposed approach. Further analysis reveals that errors tend to be in cases with disagreement between radiologists. We also note that pretraining is critical for a hybrid CNN-transformer model on this task, likely due to the small labelled dataset. Lastly, curation of temporal training data is observed to improve the classification results by .68 pp aggregated across the findings.

**III. Static tasks benefit from temporal learning:** BioViL-T broadens the range of applicable downstream tasks whilst contributing to performance on static tasks.

In this section, we demonstrate that performance improvements afforded by BioViL-T are not restricted to temporal tasks - static tasks also benefit. Below, we report results on zero- and few-shot pneumonia classification from single images, where BioViL-T establishes a new SOTA compared to prior work.

RSNA Pneumonia Detection Benchmark Classification results for train & test split of 70% - 30% respectively

| Method | % of Labels | Supervision | Acc. | F1 | AUROC |
|---|---|---|---|---|---|
| GLoRIA | ✗ | Zero-shot | 0.70 | 0.58 | - |
| BioViL | | ✗ | Zero-shot | 0.732 | 0.665 | 0.831 |
| BioViL-T | ✗ | Zero-shot | **0.805** | **0.706** | **0.871** |
| BioViL | 1% | Few-shot | 0.805 | 0.723 | 0.881 |
| BioViL-T | 1% | Few-shot | **0.814** | **0.730** | **0.890** |

We see a similar trend on the MS-CXR phrase grounding benchmark (below). This task can be solved with single images, however we show that the inclusion of the prior image (where available) does not impair the performance of BioViL-T: feature decomposition effectively preserves localised information from the current image.

MS-CXR benchmark results (5-runs with different seeds) "Multi-image" column indicates the input images used at test time.

| Method | Multi-Image | Avg. CNR | Avg. mIoU |
|---|---|---|---|
| BioViL | ✗ ✗ | 1.07 ± 0.04 | 0.229 ± 0.005 |
| + Local loss | ✗ | 1.21 ± 0.05 | 0.202 ± 0.010 |
| BioViL-T | ✗ | **1.33 ± 0.04** | **0.243 ± 0.005** |
| BioViL-T | ✓ | **1.32 ± 0.04** | **0.240 ± 0.005** |

**IV. Toward better sentence embedding quality:** Language models acquire increased sensitivity.

We hypothesise that text encoders learn temporal semantics through supervision from longitudinal image series. To verify this, RadNLI and *CXR-T* datasets are used in a zero-shot binary classification setting. Cosine similarity of sentence pair embeddings are treated as class-logits to label each pair either as paraphrase or contradiction.

Our text model is benchmarked against SOTA domain specific BERT models. The results below (*CXR-T*), collected over 4 runs, show that the proposed framework greatly increases the sensitivity of sentence embeddings to temporal content. At same time, it learns to better capture the static content (RadNLI). Here, CXR-BERT-Specialised is a text-model learnt through single-image based pretraining. Note that our text model is initialised with CXRBERT-General, illustrating the substantial increase in temporal and static sensitivity due to BioViL-T pre-training.

| Text Model | CXR-T (361 pairs) | | RadNLI (145 pairs) | |
|---|---|---|---|---|
| | Accuracy | ROC-AUC | Accuracy | ROC-AUC |
| PubMedBERT | 60.39 | .542 | 81.38 | .727 |
| CXR-BERT-G | 62.60 | .601 | 87.59 | .902 |
| CXR-BERT-S | 78.12 | .837 | 89.66 | .932 |
| BioViL-T | **87.77 ± 0.5** | **.933 ± .003** | 90.52 ± 1.0 | **.947 ± .003** |

## FINAL REMARKS

It will be appreciated that the above embodiments have been disclosed by way of example only.

More generally, according to one aspect disclosed herein, there is provided a computer-implemented method of machine learning, the method comprising: accessing a machine learning model; accessing a training data point comprising i) a present image of a target, ii) a prior image of the target captured from an earlier occasion than the present image, and iii) and a corresponding textual report on the present and prior images; operating the machine learning model to perform encoding operations comprising: - using an image encoder of the machine learning model, encoding the present image into a present image embedding, and encoding the prior image into a prior image embedding, - using a difference encoder of the machine learning model, generating a difference embedding representing a difference between the present image embedding and the prior image embedding, and - using a text encoder of the machine learning model, encoding the textual report into a text embedding; and training the machine learning model by comparing the text embedding with a temporal image embedding comprising the present image embedding and difference embedding.

In embodiments, said accessing may comprise accessing a plurality of training data points, each comprising i) a respective present image of a respective target, ii) a corresponding prior image of the respective target captured from an earlier time than the respective present image, and iii) and a respective corresponding textual report on the present and prior images. In this case the encoding operations comprise, for each of the plurality of training data points: - using the image encoder to encode the respective present image into a respective present image embedding, and to encode the respective prior image into a respective prior image embedding, - using the difference encoder to generate a respective difference embedding representing the difference between the respective present image embedding and the respective prior image embedding, and - using the text encoder to encode the respective textual report into a respective embedding; and the method further comprises the training comprises training the machine learning model over the plurality of training data points, by comparing the text embedding of each of the plurality of training data points with a respective temporal image embedding comprising the respective present image embedding and respective difference embedding.

In embodiments the present image embedding for each training data point may comprise a respective plurality of constituent present patch embeddings encoded from different patches of the respective present image, the prior image embedding for each training data point may comprise a respective plurality of constituent prior patch embeddings encoded from different patches of the respective prior image, and the text embedding for each training data point may comprise a respective plurality of constituent text-portion embeddings each being encoded from a different portion of the textual report. In this case the difference embedding for each training data point comprises a respective plurality of constituent difference patch embeddings generated based on the respective plurality of present patch embeddings and the respective plurality of prior patch embeddings; and for each training data point, the comparing comprises one or both of: - for each of the respective plurality of constituent text-portion embeddings, computing a local similarity between the constituent text-portion embedding and each of the present patch embeddings and difference embeddings of the respective temporal patch embedding, and/or - computing a global similarity between the respective plurality of text-portion embeddings and the respective temporal image embedding.

In embodiments, the difference encoder may comprise a vision transformer, and for each training data point the generating of the difference patch embeddings may comprise: - combining each of the respective plurality of present patch embeddings with a spatial embedding identifying a position of the patch from which the present patch embedding was encoded, combining each of the respective plurality of prior patch embeddings with a spatial embedding identifying a position of the patch from which prior the patch embedding was encoded, and combining each of the present and past image embeddings with a temporal embedding identifying a time of capture of the present and past image respectively, thereby resulting in a spatially and temporally encoded version of each of the present and past image embeddings, and - inputting the spatially and temporally encoded version of the present and past image embeddings into the vision transformer, thereby resulting in the respective difference embedding comprising the respective plurality of difference patch embeddings; wherein the training comprises training the vision transformer.

In embodiments the image encoder may comprise a neural network having shared weights for encoding both the present images and prior images. The neural network may comprise a convolutional neural network.

In embodiments the method may comprise, for each training data point, performing a projection such that the respective text embedding, present image embedding and difference embedding are normalized into a common latent space prior to the comparing of the respective text embedding with the respective present image embedding and difference embedding.

In embodiments, the method may further comprise, for at least one of the training data points: masking one or more parts of the respective textual report, thereby resulting in masked text; using the text encoder combined with cross-attention from the respective current image embedding and difference embedding to encode the masked text, thereby resulting in a masked-text embedding; predicting at least one of the one or more masked parts of the respective textual report using a masked language modelling, MLM, module of the machine learning model; and determining a MLM loss based on the masked-text embedding and prediction of the at least one masked part, and based thereon providing a feedback signal to bias the training of the image encoder and text encoder.

In embodiments the method may further comprise: accessing at least one partial data point comprising respective textual report and a respective present image but no prior image; performing further encoding operations comprising: - using the image encoder to encode the respective present image of the partial data point into a respective present image embedding, - using a missing image encoder of the machine learning model to generate a missing image embedding as a placeholder in lieu of a respective difference embedding, and - using the text encoder to encode the respective textual report of the partial data point into a text embedding; and training the machine learning model by comparing the respective text embedding of the partial data point with the respective present image embedding and with the missing image embedding, wherein the training comprises training the missing image encoder to learn to generate missing image embeddings to act as a placeholders for missing prior images.

In embodiments the method may further comprise, in a deployment stage following said training: receiving a query comprising at least a respective present image and a draft report comprising text describing a target shown in the respective present image; inputting the queried image into the image encoder, thereby generating a respective present image embedding; inputting at least some of the text of the draft report into the text encoder, thereby generating a respective text embedding; based on the respective present image embedding and text embedding of the query, generating a proposed update to the text of the draft report; and outputting the proposed update to a user, thereby causing the user to action a treatment on the target based on the proposed update.

In embodiments the query may further comprise a respective prior image captured from an earlier time than the respective present image, and the method may further comprise inputting the respective prior image of the query into the image encoder to generate a respective prior image embedding, wherein the generating of the proposed update is further based on the respective prior image embedding of the query.

In embodiments the query does not necessarily comprise any prior image captured from an earlier time than the respective present image.

In embodiments the method may further comprise, following said training, finetuning the model based on at least one manually-labelled series of two or more images of a target captured on different occasions.

In embodiments the target of the query may be a human or animal body or part thereof, and the treatment may comprise a medical treatment of the human or animal body or part thereof.

According to another aspect disclosed herein, there is provided a computer system comprising: processing apparatus comprising one or more processors, and memory comprising one or more memory units; wherein the memory stores code arranged to run on the processing apparatus and configured so as when run to perform any method disclosed herein.

According to another aspect disclosed herein, there is provided a computer program embodied on non-transitory computer-readable storage, configured so as when run on one or more processors to perform the method of any embodiment disclosed herein.

Other variants or use cases may become apparent to a person skilled in the art once given the disclosure herein. The scope of the present disclosure is not limited by the above-described embodiments, but only by the accompanying claims.

**Claims**

1. A computer-implemented method of machine learning, the method comprising:

   accessing a machine learning model;
   accessing a training data point comprising i) a present image of a target, ii) a prior image of the target captured from an earlier occasion than the present image, and iii) and a corresponding textual report on the present and prior images;
   operating the machine learning model to perform encoding operations comprising:

   - using an image encoder of the machine learning model, encoding the present image into a present image embedding, and encoding the prior image into a prior image embedding,
   - using a difference encoder of the machine learning model, generating a difference embedding representing a difference between the present image embedding and the prior image embedding, and
   - using a text encoder of the machine learning model, encoding the textual report into a text embedding; and

   training the machine learning model by comparing the text embedding with a temporal image embedding comprising the present image embedding and difference embedding.

2. The method of claim 1, wherein said accessing comprises accessing a plurality of training data points, each comprising i) a respective present image of a respective target, ii) a corresponding prior image of the respective target captured from an earlier time than the respective present image, and iii) and a respective corresponding textual report on the present and prior images;

   the encoding operations comprise, for each of the plurality of training data points:

   - using the image encoder to encode the respective present image into a respective present image embedding, and to encode the respective prior image into a respective prior image embedding,
   - using the difference encoder to generate a respective difference embedding representing the difference between the respective present image embedding and the respective prior image embedding, and
   - using the text encoder to encode the respective textual report into a respective embedding; and

   the training comprises training the machine learning model over the plurality of training data points, by comparing the text embedding of each of the plurality of training data points with a respective temporal image embedding comprising the respective present image embedding and respective difference embedding.

3. The method of claim 2, wherein:

   the present image embedding for each training data point comprises a respective plurality of constituent present patch embeddings encoded from different patches of the respective present image, the prior image embedding for each training data point comprises a respective plurality of constituent prior patch embeddings encoded from different patches of the respective prior image, and the text embedding for each training data point comprises a respective plurality of constituent text-portion embeddings each being encoded from a different portion of the textual report;

the difference embedding for each training data point comprises a respective plurality of constituent difference patch embeddings generated based on the respective plurality of present patch embeddings and the respective plurality of prior patch embeddings; and

for each training data point, the comparing comprises one or both of:

- for each of the respective plurality of constituent text-portion embeddings, computing a local similarity between the constituent text-portion embedding and each of the present patch embeddings and difference embeddings of the respective temporal patch embedding, and/or
- computing a global similarity between the respective plurality of text-portion embeddings and the respective temporal image embedding.

4. The method of claim 3, wherein the difference encoder comprises a vision transformer, and for each training data point the generating of the difference patch embeddings comprises:

- combining each of the respective plurality of present patch embeddings with a spatial embedding identifying a position of the patch from which the present patch embedding was encoded, combining each of the respective plurality of prior patch embeddings with a spatial embedding identifying a position of the patch from which prior the patch embedding was encoded, and combining each of the present and past image embeddings with a temporal embedding identifying a time of capture of the present and past image respectively, thereby resulting in a spatially and temporally encoded version of each of the present and past image embeddings, and
- inputting the spatially and temporally encoded version of the present and past image embeddings into the vision transformer, thereby resulting in the respective difference embedding comprising the respective plurality of difference patch embeddings;

wherein the training comprises training the vision transformer.

5. The method any of claims 2 to 4, wherein the image encoder comprises a neural network having shared weights for encoding both the present images and prior images.

6. The method of any of claims 2 to 5, comprising for each training data point, performing a projection such that the respective text embedding, present image embedding and difference embedding are normalized into a common latent space prior to the comparing of the respective text embedding with the respective present image embedding and difference embedding.

7. The method of any of claims 2 to 6, further comprising for at least one of the training data points:

masking one or more parts of the respective textual report, thereby resulting in masked text;

using the text encoder combined with cross-attention from the respective current image embedding and difference embedding to encode the masked text, thereby resulting in a masked-text embedding;

predicting at least one of the one or more masked parts of the respective textual report using a masked language modelling, MLM, module of the machine learning model; and

determining a MLM loss based on the masked-text embedding and prediction of the at least one masked part, and based thereon providing a feedback signal to bias the training of the image encoder and text encoder.

8. The method of any preceding claim, further comprising:

accessing at least one partial data point comprising respective textual report and a respective present image but no prior image;

performing further encoding operations comprising:

- using the image encoder to encode the respective present image of the partial data point into a respective present image embedding,
- using a missing image encoder of the machine learning model to generate a missing image embedding as a placeholder in lieu of a respective difference embedding, and
- using the text encoder to encode the respective textual report of the partial data point into a text embedding; and

training the machine learning model by comparing the respective text embedding of the partial data point with the respective present image embedding and with the missing image embedding, wherein the training comprises training the missing image encoder to learn to generate missing image embeddings to act as a placeholders for missing prior images.

9. The method of any preceding claim, comprising, in a deployment stage following said training:

receiving a query comprising at least a respective present image and a draft report comprising text describing a target shown in the respective present image;
inputting the queried image into the image encoder, thereby generating a respective present image embedding;
inputting at least some of the text of the draft report into the text encoder, thereby generating a respective text embedding;
based on the respective present image embedding and text embedding of the query, generating a proposed update to the text of the draft report; and
outputting the proposed update to a user, thereby causing the user to action a treatment on the target based on the proposed update.

10. The method of claim 9, wherein query further comprises a respective prior image captured from an earlier time than the respective present image, and the method further comprises inputting the respective prior image of the query into the image encoder to generate a respective prior image embedding, wherein the generating of the proposed update is further based on the respective prior image embedding of the query.

11. The method of claim 10, wherein the query does not comprise any prior image captured from an earlier time than the respective present image.

12. The method of any preceding claims, further comprising, following said training, finetuning the model based on at least one manually-labelled series of two or more images of a target captured on different occasions.

13. The method of claim 10, 11 or 12, wherein the target of the query is a human or animal body or part thereof, and the treatment comprises a medical treatment of the human or animal body or part thereof.

14. A computer system comprising:

processing apparatus comprising one or more processors, and
memory comprising one or more memory units,
wherein the memory stores code arranged to run on the processing apparatus and configured so as when run to perform the method of any preceding claim.

15. A computer program embodied on non-transitory computer-readable storage, configured so as when run on one or more processors to perform the method of any of claims 1 to 13.

# Figure 1

# Figure 2A

```
┌─────────────────────┐
│  Unsupervised pre-  │      P10
│  training of model  │ ∿╱
└─────────────────────┘
           │
           ▼
┌─────────────────────┐
│   Deployment and    │      P20
│ optional supervised │ ∿╱
│      training       │
└─────────────────────┘
```

# Figure 2B

```
┌─────────────────────┐          ┌─────────────────────┐
│ Receive input images│  R10     │  Receive text query │  R20
│                     │ ∿╱       │                     │ ∿╱
└─────────────────────┘          └─────────────────────┘
           │                                │
           ▼                                ▼
┌─────────────────────┐          ┌─────────────────────┐
│ Generate local image│  R30     │    Generate text    │  R40
│     embeddings      │ ∿╱       │     embeddings      │ ∿╱
└─────────────────────┘          └─────────────────────┘
           │                                │
           └────────────────┬───────────────┘
                            ▼
                  ┌─────────────────────┐
                  │       Align         │  R50
                  │                     │ ∿╱
                  └─────────────────────┘
                            │
                            ▼
                  ┌─────────────────────┐
                  │  Output image with  │  R60
                  │ text query matched  │ ∿╱
                  │  to region in image │
                  └─────────────────────┘
```

# Figure 2C

202

S10) Scan(s)

106

S20) 107

204

116

ML Model

User

S30) Report

S40)

S50)

206

Suggested updates

Figure 3

Existing methods · Disclosed method

(a)

202_curr

Image encoder 302

Current image

204 → Text encoder 304

Clinical report

InfoNCE affinity matrix

(c)

202_prior · Prior image (if available) · 202_curr · Current image

Image encoder 302'

204 → Text encoder 304'

Clinical report

InfoNCE affinity matrix

(b) Spatial modelling

(d) Spatiotemporal modelling

· · · · · Prior image · · · · ·

· · · · · Current image · · · · ·

"pleural fluid in the right base"

"lung nodule remains unchanged"

"pleural effusion is worsening"

EP 4 398 157 A1

Figure 4

# Figure 5

$x_{img}^{prior}$ (if available)

$x_{img}^{curr}$

302'

Multi-image encoder

510

(Cross attention to image features)

$w_{txt}^{query}$ (e.g. $w_{txt}^{prior}$)

...

500

Text decoder

...

520

<table>
<tr><td>

Europäisches<br>Patentamt<br>European<br>Patent Office<br>Office européen<br>des brevets

</td><td>

**EUROPEAN SEARCH REPORT**

</td><td>

**Application Number**

EP 23 15 0606

</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | XIAN WU ET AL: "DeltaNet:Conditional Medical Report Generation for COVID-19 Diagnosis", ARXIV.ORG, CORNELL UNIVERSITY LIBRARY, 201 OLIN LIBRARY CORNELL UNIVERSITY ITHACA, NY 14853, 12 November 2022 (2022-11-12), XP091377612, * the whole document * | 1-15 | INV. G06N3/088 G06N3/045 G16H50/20 |
| Y | SANGJOON PARK ET AL: "Self-supervised Co-learning of Uncurated Images and Reports Enables Oversight AI in Radiology", ARXIV.ORG, CORNELL UNIVERSITY LIBRARY, 201 OLIN LIBRARY CORNELL UNIVERSITY ITHACA, NY 14853, 10 August 2022 (2022-08-10), XP091352189, * the whole document * | 1-15 | |
| Y | JIANJIE LUO ET AL: "CoCo-BERT: Improving Video-Language Pre-training with Contrastive Cross-modal Matching and Denoising", ARXIV.ORG, CORNELL UNIVERSITY LIBRARY, 201 OLIN LIBRARY CORNELL UNIVERSITY ITHACA, NY 14853, 14 December 2021 (2021-12-14), XP091121125, * abstract; figures 1,2 * * Section 4 * | 8 | TECHNICAL FIELDS SEARCHED (IPC)<br><br>G06N<br>G16H |

−/−−

The present search report has been drawn up for all claims

| Place of search | Date of completion of the search | Examiner |
|---|---|---|
| The Hague | 22 May 2023 | Mariani, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 23 15 0606

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | SHRUTHI BANNUR ET AL: "Learning to Exploit Temporal Structure for Biomedical Vision-Language Processing", ARXIV.ORG, CORNELL UNIVERSITY LIBRARY, 201 OLIN LIBRARY CORNELL UNIVERSITY ITHACA, NY 14853, 11 January 2023 (2023-01-11), XP091456111, * the whole document * | 1-15 | |
| A | MENGWEI REN ET AL: "Local Spatiotemporal Representation Learning for Longitudinally-consistent Neuroimage Analysis", ARXIV.ORG, CORNELL UNIVERSITY LIBRARY, 201 OLIN LIBRARY CORNELL UNIVERSITY ITHACA, NY 14853, 9 June 2022 (2022-06-09), XP091243529, * abstract; figures 1, 2, 3 * * Sections 1, 3 * | 1-15 | |

TECHNICAL FIELDS
SEARCHED      (IPC)

The present search report has been drawn up for all claims

| Place of search | Date of completion of the search | Examiner |
|---|---|---|
| The Hague | 22 May 2023 | Mariani, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)